# EUROPEAN PATENT SPECIFICATION

(11) **EP 0 506 314 B1**
(45) Date of publication and mention of the grant of the patent: **30.01.2002**
(21) Application number: 92302459.0
(22) Date of filing: 23.03.1992
(51) Int. Cl.: C07B 37/04, C07B 63/02, C07C 13/44, C07C 15/46, C07C 2/86, C07F 7/18

(54) **Process for preparing vinylically-unsaturated compounds**
Verfahren zur Herstellung von vinyl-ungesättigten Verbindungen
Procédé pour la préparation de composés vinyliques insaturés

(30) Priority: 28.03.1991 US 677023; 28.03.1991 US 676622; 30.04.1991 US 694521; 09.10.1991 US 773472
(43) Date of publication of application: 30.09.1992
(73) Proprietor: THE DOW CHEMICAL COMPANY, Midland, Michigan 48674 (US)
(72) Inventor: DeVries, Robert A., Midland, Michigan 48640 (US); Schmidt, Gregory F., Midland, Michigan 48640 (US); Frick, Hughie R., Deceased (US); Mendoza, Abel, Midland, Michigan 48640 (US)
(74) Representative: Burford, Anthony Frederick

(56) References cited:
- EP-A- 0 062 608
- US-A- 3 922 299
- US-A- 4 724 260
- US-A- 4 812 588
- US-A- 4 831 172
- JOURNAL OF ORGANIC CHEMISTRY vol. 43, no. 12, 1978, WASHINGTON D.C.,US pages 2454 - 2456 R F HECK ET AL 'Palladium catalyzed arylation of ethylene'

## Description

This invention relates to an improved process for the synthesis of vinylically-unsaturated compounds by reaction between halogenated organic compounds and vinylically-unsaturated precursor compounds.

The palladium-catalyzed vinylation of organic halides has been reviewed by Heck, Organic Reactions, vol. 27 (1982), beginning at page 345. Process conditions, recited at page 360, do not require the use of a solvent, although an organic amine can apparently function as a solvent. Other solvents used include acetonitrile, methanol, dimethylformamide, N-methylpyrrolidinone and hexamethyl phosphoramide. It is stated that vinylic substitution is quite insensitive to impurities in the reactants, so highly purified reagents are not necessary and that small amounts of water also do not interfere.

Heck, US-A-3,922,299, teaches that the reaction can be carried out with or without a solvent. Suggested solvents include acetonitrile, tetrahydrofuran or excess olefin.

Vinylically-unsaturated organosilicon compounds are useful as adhesion promoters, particularly for electronic applications. Formulators of adhesives for electronic utilization generally require vinylically-unsaturated organosilicon compounds, containing very low levels of impurities, particularly heavy metal and other inorganic impurities. Organosilicon compounds frequently contain heavy metals, halogens, alkali metals and phosphorus. Any of these materials, in amounts greater than 1 to 10 ppm, can cause objectionable properties in adhesive formulations, containing vinylically-unsaturated organosilicon compounds.

The Heck vinylation reaction has been used to vinylate various kinds of compounds, including vinylation using vinylically-unsaturated organosilicon compounds. The preparation of polysiloxane-bridged bisbenzocyclobutene monomers has been recited by Gros, US-A-4,759,874, and Schrock, US-A-4,812,588. Schrock '588 discloses chromatography of a product from 4-bromobenzocyclobutene and 1,1'-divinyltetramethyldisiloxane over silica gel. Other silane-containing compounds have been synthesized by Hahn et al., US-A-4,831,172. Similar synthesis of benzocyclobutene compounds is disclosed by Kirchhoff, US-A-4,540,763. Kirchhoff et al., US-A-4,724,260, have prepared an acetylenically-unsaturated organosilicon compounds from 4-bromobenzocyclobutene and trimethyl silylacetylene, using bistriphenylphosphine palladium (II) chloride and cuprous iodide catalysts and triethylamine as hydrogen halide acceptor.

EP-A-0062608 (corresponding to US-A-4,918,215) discloses the preparation of certain 4-halo-4'-vinylstilbene derivatives by (a) reaction of a 4-halostyrene with a 4-carbonyl halide-styrene derivative in the presence of a phosphorus-free labile zerovalent palladium catalyst formed *in situ* or (b) reaction of a 4,4'-dihalostilbene with a vinyl compound in the presence of an arsenic or phosphorus-containing palladium catalyst. The reactions are preferably conducted in an organic solvent but can be conducted in a two-phase system of water and a water-immiscible solvent.

Plevyak & Heck (J. Org. Chem., 43 (1978) 2454-2456 discloses the preparation of styrene derivatives and 3-vinylpyridine by the palladium tri(o-tolyl)phosphine catalyzed reaction of ethylene with aryl bromides and 3-bromopyridine respectively. Acetonitrile or dimethylformamide were used as the reaction solvent.

Known processes for reaction between halogenated organic compounds and vinylically-unsaturated precursor compounds have been carried out in organic solvents.

This invention provides a process for preparing a vinylically-unsaturated product compound, comprising reacting a halogenated organic compound with a vinylically-unsaturated precursor compound in the presence of (a) a homogeneous zerovalent palladium catalyst complex, (b) a hydrogen halide acceptor and (c) a diluent, wherein the diluent is water or an aqueous solution containing up to 95 percent by volume of an organic solvent and providing sufficient water to dissolve hydrohalide salt formed during the reaction.

In a preferred embodiment, the crude product mixture is treated with a peroxide to remove organophosphine or organoarsine present in the mixture.

The reaction between halogenated organic compounds and vinylically-unsaturated precursor compounds is carried out in the presence of a zerovalent palladium catalyst complex. The catalyst complex can be added to the reaction mixture or can be formed in the reaction mixture. Representative preformed catalyst complexes include tetrakis-(triphenylphosphine)palladium (0), tris(dibenzylideneacetone)dipalladium (0) with triphenylphosphine and dichloro(triphenylphosphine)-palladium (II). Advantageously, the zerovalent palladium catalyst complex is insoluble in water. It is not necessary for it to contain a sulfonate group to make it miscible with the aqueous phase. Accordingly, in preferred embodiments, the zerovalent palladium catalyst complex is insoluble in water.

The catalyst complex can be prepared in the reaction mixture, generally by reaction between a palladium (II) compound and a trivalent organophosphorus or organoarsenic compound.

The reaction between the vinylically-unsaturated precursor compound and halogenated organic compound, in the presence of a representative palladium complex, can be represented by the general equation: 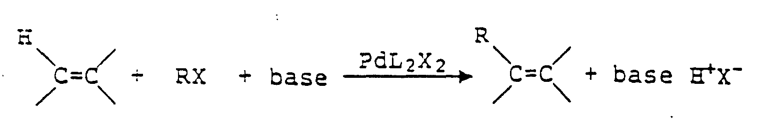 wherein R is aryl, heterocyclic, vinylic or benzyl and X is bromo, iodo or, rarely, chloro. L represents a ligand, which is a trivalent organophosphorus or organoarsenic compound. The base in the equation corresponds to the hydrogen halide acceptor.

Representative ligands in the Pd catalytic complex include, but are not limited to, triphenylarsine, triphenyl arsenite, tri-(n-butyl)phosphine, diphenylmethylphosphine, diphenylmethoxyphosphine, trimethyl phosphite, triphenylphosphine, triethylphosphine, phenyldi-(n-butoxy)phosphine, tris-(p--anisyl)phosphine tris-(o-tolyl)phosphine and tris-(o--tolyl)phosphite.

Palladium can be introduced into the reaction mixture in the form of a salt, such as the acetate or chloride. It is postulated that the catalyst complex should contain two phosphine or arsine ligands per palladium atom.

Catalysts, or catalytic complexes, formed from palladium (II) acylates, particularly Pd(II) acetate, and triaryl phosphines have been found to be particularly preferred for the practice of this invention. Particularly preferred triaryl phosphines are triphenylphosphine and tris-(o-tolyl)phosphine.

Most preferred catalysts are those obtained from Pd(II) acetate and tris-(o-tolyl)phosphine.

The molar ratio of palladium (II) compound to phosphorus or arsenic ligand suitably can be varied from 1:1 to 1:100. It is preferred to operate at ratios of from 1:2 to 1:10, particularly when a catalyst from Pd(II) acetate and tris(o-tolyl)phosphine is used.

The amount of catalyst complex usually is varied from 0.1 mol to 0.00001 mol (as Pd) per mol of halogenated organic compound in the reaction mixture. Preferably, the catalyst level is 0.01 to 0.00001 mol (as Pd) per mol of halogenated organic compound.

It has been found that maintaining a molar range of from 1:3 to 1:20 and preferably from 1:4 to 1:10 of palladium to organophosphine or organoarsine will result in the more efficient use of palladium in the conversion of halogenated organic compound to the vinylically unsaturated product compound. Use of the palladium to ligand ratio in this range permits reasonable conversion rates at a halogenated organic compound to palladium molar ratio of more than 200:1 or even 400:1. Preferably, the halogenated organic compound to palladium molar ratio is less than 600:1. Reasonable conversion rates include 90 percent conversion of the halogenated organic compound in less than 20 hours and more preferably in less than 8 hours. Organic hydrogen halide acceptors, used in the practice of this invention, usually are secondary or tertiary amines. Representative organic hydrogen halide acceptors include, but are not limited to, trimethyl-amine, triethylamine, methylethylamine, diethyl-n-butylamine, triisobutylamine, tri-n-butyl-amine, diisopropylamine, triisopropylamine, N, N, N', N'-tetramethyl-ethylene diamine, N-methylcyclohexylamine, N,N-diethyl-cyclohexylamine, N,N-diethylaniline, N,N-dimethyl-aniline, N-methyltoluidine, pyridine, quinoline, the lutidines, N-methylpiperidine and N-methylpyrrole.

Preferred organic hydrogen halide acceptors are tertiary amines, particularly those represented by the formula R₁R₂R₃N, wherein each of R₁, R₂ and R₃ is selected independently from straight-chain and branched-chain alkyl of 1 to 8 carbon atoms and cycloalkyl. Most preferably, the organic hydrogen halide acceptor is triethylamine.

The molar ratio of organic hydrogen halide acceptor to halogenated organic compound suitably can be varied from 1:1 to 10:1. Using greater excesses of organic hydrogen halide acceptor is not particularly advantageous. It has been found that excellent results are obtained using 1 to 2 mols of organic hydrogen halide acceptor per mol of halogenated organic compound. Therefore, preferred ratios of organic hydrogen halide acceptor to halogenated organic compound are from 1:1 to 2:1.

Inorganic hydrogen halide acceptors usually are selected from salts of weak acids and strong bases or corresponding oxides or hydroxides, particularly of the Group I alkali metals and Group II alkaline earth metals. Inorganic hydrogen halide acceptors accordingly include salts, oxides and hydroxides of lithium, sodium, potassium, cesium, magnesium, calcium, strontium and barium.

Preferred inorganic hydrogen halide acceptors are selected from alkali metal hydroxides, carbonates and acylates, particularly acetates or propionates. Most preferred are sodium or potassium acetate.

The molar ratio of inorganic hydrogen halide acceptor to halogenated organic compound suitably can be varied from 1:1 to 10:1. Using greater excesses of inorganic hydrogen halide acceptor is not particularly advantageous. It has surprisingly, been found that excellent results are obtained using 1.5 to 2:1 molar ratios of inorganic hydrogen halide acceptor to halogenated organic compound. Therefore, preferred ratios of inorganic hydrogen halide acceptor to halogenated organic compound are from 1:1 to 2:1.

An organic hydrogen halide acceptor may optionally be used in conjunction with an inorganic hydrogen halide acceptor in the practice of this invention.

When used in conjunction with the inorganic hydrogen halide acceptor, the amount of optional organic hydrogen halide acceptor used is normally 0.5 to 2.5 moles per mol of inorganic hydrogen halide acceptor. It is frequently preferred, owing to flammability, odor, cost and environmentally objectionable properties of organic hydrogen halide acceptors, to use no organic hydrogen halide acceptor in the process of this invention when using an aqueous diluent.

The diluent is water or an aqueous solution, containing up to 95 percent by volume of organic solvent. Preferably, the diluent contains 25 percent by volume or more water and most preferably 70 percent by volume or more water.

Preferably, the diluent water is present in a quantity sufficient to dissolve any inorganic salts formed during the reaction. When water is used as the diluent, the amount of water preferably exceeds 50 percent by weight of the combined organic reactants and organic hydrogen halide acceptor. Combined organic reactants means the combined weights of halogenated organic compound and vinylically-unsaturated precursor compound. More preferably, the amount of water exceeds 100 percent by weight of the combined organic reactants and organic hydrogen halide acceptor. Preferably, the amount of water is less than 500 percent by weight of the combined organic reactants and organic hydrogen halide acceptor. The amount of water is selected, within these limits, so as to be sufficient to dissolve the hydrohalide salt, formed during the reaction. Use of an amount of water, sufficient to dissolve salts formed by the reaction, greatly facilitates isolation and purification of the reaction products.

The use of an aqueous solvent in the process does not adversely affect the yield or product distribution. Use of a diluent, containing significant amounts of water prevents formation of intractable salt accumulations in the reactor and on the stirrer. A reaction medium, from which byproducts do not accumulate on the walls of the reactor or stirrer, provides for more efficient heat transfer and for better agitation than possible when salt-cake formation occurs.

In some cases, phase separation into an aqueous salt-containing layer and an organic product-containing layer occurs. Separation of the reaction mixture into a two-phase system is very desirable and facilitates removal of the by-product salt and isolating the desired product from the organic phase.

Aqueous solutions of organic solvents, also used as diluents, preferably contain up to 90 percent by volume of organic solvent. Organic solvents suitably can be selected from nitriles, alcohols, ketones, linear or cyclic saturated esters, N,N-dialkylformamides, N-alkylpyrrolidinones, alkoxyalkanols, glycol ether, dioxane, tetrahydrofuran, tetrahydropyran or hexaalkylphosphoramides. It will be understood that the foregoing organic solvents are representative of water-miscible solvents, acceptable for use in the process of this invention.

Suitable nitriles include, but are not limited to, acetonitrile, propionitrile, butyronitrile, and higher aliphatic nitriles, as well as benzonitrile, tolylnitrile, and methoxybenzonitrile. A preferred nitrile solvent is acetonitrile.

Alcohols which can be used in the aqueous diluent solutions include alkanols of 1 to 8 carbon atoms, including the various isomeric forms.

Esters useful as diluent solutions include linear or cyclic saturated esters, for example, ethyl acetate, methyl propionate, isopropyl butyrate, caprolactone and butyrolactone.

Ketones, useful in the aqueous diluent solutions, include acetone, methyl ethyl ketone, methyl isopropyl ketone, and similar compounds.

Of the various N,N-dialkylformamides which can be used in the aqueous diluent, N,N-dimethylformamide is most preferred.

N-Methylpyrrolidinone is preferred among the various N-alkylpyrrolidinones.

Alkoxyalkanols suitable for use in aqueous diluents include those of up to 10 carbon atoms, for example, ethylene glycol monomethyl ether, propylene glycol monoethyl ether, ethylene glycol monobutyl ether and propylene glycol monoisopropyl ether.

Glycol ethers, including ethylene glycol dimethyl or diethyl ethers, corresponding propylene glycol ethers, or diethylene glycol or triethylene glycol diethers can also be used.

Of the hexaalkylphosphoramides which can be used in the aqueous diluents, hexamethylphosphoramide is preferred.

Preferred aqueous diluents are those containing 10 to 90 percent by volume of N,N-dimethylformamide or N-methylpyrrolidinone. Most preferred diluents are aqueous solutions containing 30 to 70 percent by volume of dimethylformamide or N-methylpyrrolidinone.

Most preferably, the process of this invention is one wherein the diluent is an aqueous solution of 30 to 70 percent by volume of N,N-dimethylformamide or N-methylpyrrolidinone and the diluent is present in an amount equal to at least 70 percent by weight of combined halogenated organic compound, vinylically-unsaturated precursor compound and the organic hydrogen halide acceptor, if present.

Halogenated organic compounds, useful as starting materials for the process of this invention, include mono- and polycyclic, substituted or unsubstituted carbocyclic or heterocyclic aromatic bromo and iodo compounds and, rarely, chloro compounds. Aryl iodides may react in the presence of Pd(0), without a phosphine or arsine ligand. The reactive halogenated organic compounds can be classified broadly as aryl halides, benzyl halides or vinylic organic halides. It will be understood that selection of halogenated aromatic compounds is limited to those which are inert to undesired side reactions under the reaction conditions used and that this can be determined by routine experimentation.

Benzyl halides include substituted and unsubstituted benzyl chlorides, bromides and iodides. The benzyl chlorides are sufficiently reactive to add to a vinylically-unsaturated precursor compound. Benzyl chlorides and halides are preferred reagents for this synthesis. Substituents on the aromatic ring of the starting benzyl halide include straight- and branched-chain alkyl, alkoxy, nitro, cyano, hydroxy, keto, amide, carboxy, dialkylamino and sulfone groups. It will be understood that 1-halobenzocyclobutenes represent a type of benzyl halide.

Vinylic halides, useful in the practice of this invention include, for example 1- or 2-iodoalkenes and 1- or 2-bromoalkenes of the formulas CH₂=CXR or CHX=CHR, wherein R is alkyl or aryl and X is I or Br.

Aromatic halides include substituted and unsubstituted aryl bromides and iodides. Aryl chlorides generally are unreactive under the conditions used. Substituents on the aromatic ring or rings can include straight- and branched-chain alkyl, alkoxy, nitro, cyano, hydroxy, ketone, amide, carboxy, dialkylamino or sulfone groups. Aromatic halides include both monocyclic and polycyclic aromatic halides.

Representative heterocyclic reactants include, but are not limited to, bromofuran, bromopyridine, bromo-N-methylpyrrole, iodofuran and iodolutidine.

Preferably, the halogenated organic compound used as feed is selected from bromo- or iodo- mono- or polycyclic substituted or unsubstituted carbocyclic or heterocyclic compounds or substituted or unsubstituted benzyl chlorides or bromides. Bromo compounds are most preferred as starting materials.

Substituents on substituted halogenated carbocyclic aromatic compounds are preferably selected from alkyl of 1 to 6 carbon atoms, alkoxy of 1 to 6 carbon atoms, chloro or cyano. It will be appreciated that alkyl and alkoxy substituents can be straight-chain or branched-chain.

A class of preferred reactants includes bromo- or iodo- benzocyclobutenes, as disclosed by Gros, US-A-4,759,874 supra. Most preferably, the reactant is a brominated benzocyclobutene, represented by the formula 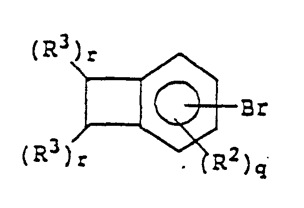 wherein R² is alkyl of 1 to 6 carbon atoms, acyloxy of 1 to 6 carbon atoms, alkoxy of 1 to 6 carbon atoms, nitro or chloro; each R³ is independently alkyl of 1 to 6 carbon atoms, aryl, nitro, chloro or cyano; q is 0, 1, 2 or 3 and each r is independently 0 or 1.

Brominated benzocyclobutenes can be prepared as recited by Liu, US-A-4,822,930. Most preferably, 4-bromo-benzocyclobutene is used in processes of this invention. This starting material is obtained in high purity by distilling materials in accordance with Liu '930.

It will be understood that "benzocyclobutene" is an art-recognized term for a cyclobutarene compound, Liu '930. Cyclobutarene compounds are compounds, containing at least one aromatic ring, which is fused to one or more substituted or unsubstituted cyclobutene ring. An aromatic ring contains (4N + 2)n electrons, as described in Morrison and Boyd, Organic Chemistry, third edition (1973). In the numbering system for benzocyclobutenes, the 1- and 2-positions are in the cyclobutene ring. The 3- and 6-positions are in an aromatic ring, adjacent to the cyclobutene ring. The 4- and 5--positions are meta- to the cyclobutene ring. Benzocyclobutenes are formally identified as derivatives of bicyclo[4.2.0] octa-1,3,5-triene. Correlations between various representative names and structures are given in Table 1.

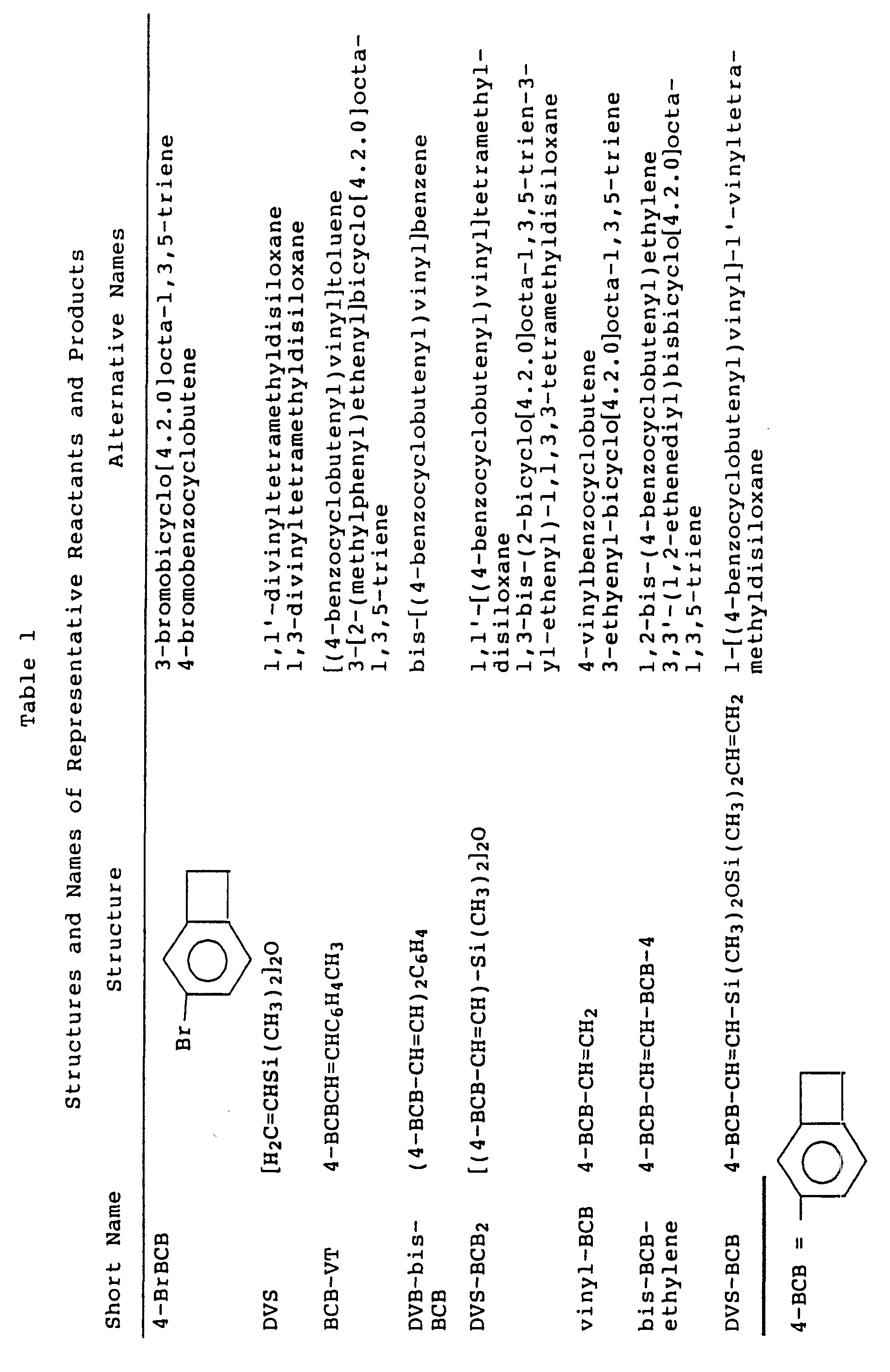

Preferred halogenated organic compounds include brominated and iodinated alkylbenzenes and alkylnapthalenes or corresponding alkoxy compounds, wherein alkyl and alkoxy are of 1 to 6 carbon atoms and can be of any isomeric structure. A particularly preferred halogenated organic compound is of the formula ArBr, wherein Ar is substituted or unsubstituted monocyclic aromatic. Most preferred as a halogenated organic compound is that wherein Ar is phenyl or o-, m- or p-tolyl, or a mixture thereof. The use of o-alkylbromo-benzenes, which react with various olefins to provide a feasible process for making hitherto hard-to-synthesize o-vinylalkylbenzenes, is particularly preferred.

Also preferred, as halogenated organic compounds are substituted or unsubstituted benzyl chlorides or bromides, particularly those wherein the substituent is alkyl or alkoxy of 1 to 6 carbon atoms.

Vinylic iodides or bromides are also preferred.

Vinylically-unsaturated precursor compounds employed in the process of this invention can be selected from three general classes of compounds:
(a) a vinyl, allyl or methallyl organosilicon compound;
(b) hydrocarbon compounds, including vinyl, allyl and methallyl hydrocarbons, of various degrees of substitution; and
(c) compounds containing vinyl, allyl or methallyl moieties and one or more of oxygen, nitrogen, phosphorus or sulfur atoms, or a combination thereof.

Vinylically-unsaturated organosilicon precursor compounds employed in the process of this invention can be selected from those containing vinyl, allyl, ethynyl or methallyl moieties. The process of this invention is applicable to vinylically-unsaturated organosilicon compounds, regardless of their hydrolytic stability.

Organosilicon compounds are commercially available. A representative source is Petrarch Systems, Inc., Bartram Road, Bristol, PA, 19007. See, for example, Petrarch's "Silicon Compounds Register and Review," (1987), page 114, which recites the availability of bis(dimethyl-amino)methylvinylsilane, 1-bromo-vinyltrimethylsilane, tert-butyldimethylvinylsilane, divinyldimethylsilane, 1,3-divinyl-1,3-diphenyl-1,3-dimethyldisiloxane, 1,3-divinyl-1,3-diphenyl-1,3-dimethyldisilazane, 1,3-divinyl-1,1,4,4-tetramethyldisilylethylene, phenyldimethylvinylsilane, phenylmethylvinylsilane, polyvinylmethylsiloxane, 1,1,3,3-tetravinyldimethyldisiloxane, tetravinylsilane, 1,3,5,7-tetramethylcyclotetrasiloxane, triphenylvinylsilane, tris(vinyldimethylsiloxy)methylsilane, tris(vinyldimethylsiloxy)methylsilane, trivinylmethylsilane and 1,3,5-trivinyl--1,1,3,5,5-pentamethyltrisiloxane, any of which is exemplary of hydrolytically-stable organosilicon compounds, useful in the practice of this invention. Preferred organosilicon compounds include those containing vinyl, allyl or methallyl function, bonded to silicon. Trialkylvinylsilanes, wherein alkyl is of 1 to 6 carbon atoms, are preferred.

A further group of preferred organosilicon compounds are di- and higher polysiloxanes, represented by the formula 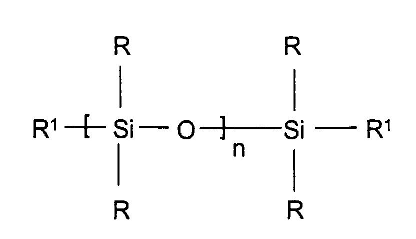 wherein each R is independently alkyl of 1 to 6 carbon atoms, cycloalkyl, aralkyl or aryl; each R¹ is independently vinyl, allyl or methallyl; and n is an integer from 1 to 4500.

Most preferably, R¹ is vinyl and each R is methyl, ethyl or phenyl. Preferred siloxanes are those wherein n is 2 to 10. Several of these compounds are available from Petrarch Systems, above. A most preferred member of this group of compounds is 1,1'-divinyltetramethyldisiloxane, represented by the formula [CH₂=CHSi(CH₃)₂]₂O.

Hydrocarbon vinylically-unsaturated precursor compounds useful in the process of this invention include compounds having a vinyl, allyl or methallyl function, whether substituted or unsubstituted by another hydrocarbon function. Compounds containing a plurality of vinyl, allyl or methallyl functions can be used, including those wherein the unsaturated bonds are conjugated. Representative vinylically-unsaturated hydrocarbon compounds for the purposes of this invention include, but are not limited to, ethylene, propylene, 1-butene, 2-butene, 2-methyl-1-propene, 1,3-butadiene, 2-methyl-1,3-butadiene, 1,4-pentadiene, 1,5-hexadiene, 3-methyl-1-butene, styrene, substituted styrenes, divinyl-benzenes, diallylbenzenes, di(methylallyl)-benzenes, 1- and 2-vinylnaphthalene and substituted vinylnaphth-alenes, vinylcyclohexane, stilbene, vinylcyclopentane, allylcyclohexane, methallylcyclohexane and substituted cyclopentanes and cyclohexanes. Substituents include alkyl and aryl groups, for example, alkyl of 1 to 6 carbon atoms, phenyl, tolyl and xylyl.

Preferred vinylically-unsaturated precursor compounds useful as starting materials for the process of this invention include ethylene, styrene, various vinyltoluene or divinyl-benzene isomers, including mixtures of isomers.

It will be understood that the process of this invention contemplates stepwise reaction between several moles of halogenated organic compound per mole of vinylically-unsaturated precursor compound. For example, 4-bromobenzocyclobutene (4-BrBCB) can be reacted with ethylene to produce (4-benzocyclobutenyl)ethylene, 1,2-bis-(4-benzocyclobutenyl)-ethylene and 1,1,2-tris-(4-benzocyclobenzocyclobutenyl)-ethylene.

Intermediate reaction products, for example, stilbene, can be reacted with additional halogenated organic compounds. Stilbene can be reacted with 4-BrBCB to produce 1,2-diphenyl-1,2-bis-(4-benzocyclobutenyl)-ethylene. Reacted with 2-bromotoluene, ethylene could produce o,o'-dimethylstilbene.

Vinylically-unsaturated precursor compounds include those containing as a hetero atom one or more of oxygen, nitrogen, phosphorus or sulfur, or a plurality thereof. These compounds include, but are not limited to acrylate and methacrylate esters, acrolein, methacrolein, ethacrolein, crotonaldehyde, vinyl acetate, vinyl octoate, vinyl propionate, vinyl versatate, vinyl laurate, allyl acetate, methallyl acetate, allyl versatate, methallyl laurate, allyl stearate, nitroethylene, nitropropylene, nitrostyrene, nitro-alpha-methylstyrene isomers, methyl vinyl ether, ethyl vinyl ether, stearyl vinyl ether, isostearyl vinyl ether, allyloxymethane, allyloxyethane, methallyloxypropane, phenyl vinyl ether, allyloxybenzene, acrylonitrile, methacrylonitrile, acrylamide, methacrylamide, N,N-diethylacrylamide, vinyl diethyl phosphite, divinylmethylphosphine, methyl vinyl thioether, vinylphosphonic acid and allylphosphonic acid.

Preferred O, S, N or P compounds include acrylonitrile or methacrylonitrile and further include acrylate or methacrylate esters, wherein the alcohol moiety is alkyl of 1 to 30 carbon atoms or substituted or unsubstituted mono- or bicyclic aryl.

Most preferred product, obtained by the process of this invention, are those prepared from:
(a) 4-bromobenzocyclobutene and a mixture of m- and p-vinyltoluene;
(b) 4-bromobenzocyclobutene and 1,1'-divinyltetramethyldisiloxane;
(c) 4-bromobenzocyclobutene and divinylbenzene
(d) ethylene and o-bromotoluene
(e) 4-bromobenzocyclobutene and ethylene and
(f) 4-bromobenzocyclobutene and styrene.

The molar ratio of halogenated organic compound to vinylically-unsaturated precursor compound can be determined by routine experimentation. Generally, molar ratios of 0.5:1 to 1.5:1 will be preferred for the synthesis of monoadducts. Higher molar ratios of halogenated organic compound to vinylically-unsaturated precursor will be employed when higher adducts are being prepared.

The temperature at which the process of this invention is performed can be from about room temperature to the temperature at which the starting materials or products decompose or polymerize. Elevated temperatures are normally preferred. It has been found that heating under reflux, generally at 80°C to 120°C, usually permits a reasonable reaction rate. The temperature conditions for a given set of reactants and diluent can readily be ascertained by routine experimentation.

For certain applications, the compounds prepared by the process of this invention are acceptable, without further purification. For example, reaction products from halogenated aromatic compounds and ethylenically-unsaturated hydrocarbons or compounds containing N, O, S or P can be used without purification more stringent than distillation or crystallization, as may be apparent to a person skilled in the art.

Products with extremely low ionic impurity levels are required when the products are being used, for example, as adhesion promoters for electronic devices. Adhesion promoters must be free of significant levels of phosphorus impurities. It has been found that treating a crude reaction mixture with a peroxide is effective to oxidize phosphine residues to a corresponding phosphine oxide. When organoarsines are used in the catalyst complex, conversion to a corresponding arsine oxide is accomplished in the same way.

Aqueous hydrogen peroxide can be used for this purpose. The crude product is treated with aqueous hydrogen peroxide at ambient or somewhat elevated temperatures for a time sufficient to oxidize residual phosphine to phosphine oxide. The use of aqueous hydrogen peroxide is preferred when the vinylically unsaturated product compound is relatively stable to hydrolysis.

Preferred organic peroxides include hydroperoxides, peresters and peracids, of which tert-butyl hydroperoxide, cumeme hydroperoxide, perbenzoic acid, m-chloroperbenzoic acid, monoperphthalic acid and peracetic acid are representative. Tert-Butyl hydroperoxide is preferred. Treatment with an organic peroxide can be done at ambient temperature or at temperatures up to 80°C to 90°C.

The mixture being treated with a peroxide can be the crude reaction mixture, containing the aqueous diluent. The mixture can contain an additional organic solvent, for example, methanol, ethanol or heptane.

The amount of peroxide is at least that necessary to oxidize any organophosphine or organoarsine to a corresponding arsine oxide or phosphine oxide. The peroxide can be added incrementally, either in portions or using a metering pump to add a solution of the peroxide. It will be understood that it may be preferable to avoid an exothermic reaction during the treatment with a peroxide and that the rate at which peroxide is added can be determined by routine experimentation.

The mixture, containing the peroxide, is stirred at room temperature or at an elevated temperature, up to the boiling point of the solvent or diluent in the mixture until conversion to phosphine or arsine oxide is complete. The treatment with peroxide is conveniently carried out under ambient pressure. However, the reaction can be carried out in a pressure vessel at pressures, greater than atmospheric. The optimum pressure and temperature conditions for use with a given reaction mixture and selected peroxide can be determined by routine experimentation.

The treated product can be filtered, if necessary, to remove any solid residues resulting from the treatment. Solvent can be removed from the mixture at this point, or the solution can be applied directly to a packed column of silica gel or alumina.

Most preferably a solution of the product mixture is applied to the column and elution is carried out using a hydrocarbon solvent, such as heptane or petroleum ether. Solvent is removed from the eluate by evaporation using conventional techniques. The amount of silica gel or alumina used to pack the chromatography column can be determined by routine experimentation.

It is preferred to purify the crude reaction products by chromatography over silica gel or alumina. This accomplishes removal of palladium residues and, when used after treatment of a crude product with a peroxide, also removes phosphine oxide.

When ultrapure, ionic-free, reaction products are required, as for adhesion promoters in electronic applications, it is preferred to treat crude reaction products with aqueous hydrogen peroxide or tert-butyl hydroperoxide and to chromatograph the crude products over silica or alumina. These purification steps can be carried out in either order.

Silica gels and alumina, suitable for chromatography, are commercially available, for example, Fisher basic alumina (80 to 200 mesh (175-75 µm), Brockman Activity 1), Baker chromatography grade silica gel (80 to 230Å; 8-23 nm) and Davison Chemical chromatographic grade 62 silica (60 x 200 mesh; 250 x 75 µm). Standard techniques for packing the chromatography column can be used.

Vinylically-unsaturated product compounds treated with a peroxide and then by chromatography over silica gel or alumina have very low inorganic impurity levels, for example, below 1 ppm of Pd or P.

Alternatively, the impure vinylically-unsaturated product compounds can be chromatographed over silica gel or alumina and then treated with a peroxide. An additional filtration step of treatment by column chromatography may be required to reduce impurities to acceptable levels.

The compounds prepared by the process of this invention have a variety of utilities. o-Vinyltoluene is an ethylenically-polymerizable monomer, useful for making resins. Derivatives of benzocyclobutenes polymerize thermally by formation of o-xylylene moieties, which can undergo Diels-Alder condensation reactions with mono-enes and provide a route to polymers with high temperature stability. Benzocyclobutenes can also act as crosslinking agents in polymers, as disclosed by Wong (US-A-4,622,375).

Accordingly, preferred processes of this invention are those wherein:
(a) the halogenated organic compound is a bromo or iodo mono- or polycyclic substituted or unsubstituted carbocyclic or heterocyclic compound or a substituted or unsubstituted benzyl chloride, bromide or iodide;
(b) the halogenated organic compound is a brominated benzocyclobutene represented by the formula 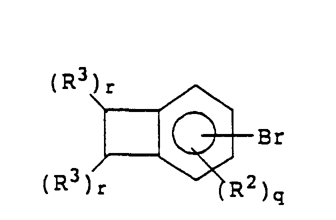 wherein R² is alkyl of 1 to 6 carbon atoms, alkoxy of 1 to 6 carbon atoms, trifluoroacetoxy, acyloxy of 1 to 6 carbon atoms, nitro or chloro; each R³ is alkyl of 1 to 6 carbon atoms, aryl, nitro, chloro or cyano; q is 0, 1, 2 or 3 and each r is independently 0 or 1;
(c) the halogenated organic compound is 4-bromobenzocyclobutene;
(d) the halogenated organic compound is of the formula ArBr, wherein Ar is phenyl or o-, m- or p-tolyl;
(e) the halogenated organic compound is a substituted or unsubstituted benzyl bromide or chloride;
(f) the halogenated organic compound is a vinylic bromide;
(g) the vinylically-unsaturated precursor compound is a vinyl, allyl or methallyl organosilicon compound, including each of (a)-(f);
(h) the vinylically-unsaturated precursor compound is a trialkylvinylsilane, wherein alkyl is of 1 to 6 carbon atoms, including each of (a)-(f);
(i) the vinylically-unsaturated precursor compound is of the formula 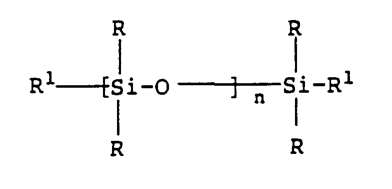 wherein each R is independently alkyl of 1 to 6 carbon atoms, cycloalkyl, aralkyl or phenyl; each R¹ is independently vinyl, allyl or methallyl and n is an integer from 1 to 4500, including each of (a)-(f);
(j)the vinylically-unsaturated precursor compound is [CH₂=CH-Si(CH₃)₂]₂O, including each of (a)-(f);
(k)the vinylically-unsaturated precursor compound is a hydrocarbon, including each of (a)-(f);
(l)the vinylically-unsaturated precursor compound is ethylene, including each of (a)-(f)
(m)the vinylically-unsaturated precursor compound is styrene, including each of (a)-(f);
(n)the vinylically-unsaturated precursor compound is o-, m- or p-vinyltoluene or a mixture thereof, including each of (a)-(f);
(o)the vinylically-unsaturated precursor compound is o-, m- or p-divinylbenzene or a mixture thereof, including each of (a)-(f);
(p)the vinylically-unsaturated precursor compound contains one or more of an oxygen atom, a nitrogen atom, a sulfur atom or a phosphorus atom or a combination thereof, including each of (a)-(f);
(q)the vinylically-unsaturated compound is acrylonitrile or methacrylonitrile, including each of (a)-(f);
(r)the vinylically-unsaturated precursor compound is an acrylate or methacrylate ester, wherein the alcohol moiety is alkyl of 1 to 30 carbon atoms or substituted or unsubstituted mono- or bicyclic aryl, including each of (a)-(f);
(s)the vinylically-unsaturated product compound is a reaction product of 4-bromobenzocyclobutene and m- or p-vinyltoluene or a mixture of the two;
(t)the vinylically-unsaturated product compound is a reaction product of 4-bromobenzocyclobutene and 1,1'-divinyl-tetramethyldisiloxane;
(u)the vinylically-unsaturated product compound is a reaction product of 4-bromobenzocyclobutene and divinylbenzene;
(v) the vinylically-unsaturated product compound is a reaction product of ethylene and o- or p-bromotoluene;
(w) the vinylically-unsaturated product compound is a reaction product of ethylene and 4-bromo-benzocyclobutene;
(x) the vinylically-unsaturated product compound is a reaction product of styrene and 4-bromo-benzocyclobutene;
(y) the catalyst is formed from a palladium (II) acetate and a triaryl phosphine, including each of (a)-(x);
(z) the catalyst is formed from a palladium (0) complex and a triarylphosphine, including each of (a)-(x);
(aa) the catalyst is formed from palladium (II) acetate and tris-(o-tolyl)phosphine, including each of (a)-(x);
(ab) the organic hydrogen halide acceptor is a secondary or tertiary amine, including each of (a)-(aa);
(ac) the organic hydrogen halide acceptor is represented by the formula R₁R₂R₃N and each of R₁, R₂ and R₃ is selected independently from straight-chain and branched-chain alkyl of 1-8 carbon atoms and cycloalkyl, including each of (a)-(aa);
(ad) the organic hydrogen halide acceptor is triethylamine, including each of (a)-(aa);
(ae) the inorganic hydrogen halide acceptor is an alkali metal hydroxide, carbonate, acetate or propionate, including each of (a)-(aa);
(af) the inorganic hydrogen halide acceptor is sodium acetate, including each of (a)-(aa);
(ag) the inorganic hydrogen halide acceptor is sodium acetate, including each of (a)-(aa);
(ah) an extra organic hydrogen halide acceptor is included with the inorganic hydrogen halide acceptor, including each of (a)-(aa);
(ai) the extra organic hydrogen halide acceptor is triethylamine, including each of (a)-(aa);
(aj) wherein the diluent is water, including each of (a)-(ai);
(ak) the diluent is water and is present in amount exceeding 100 percent by weight of combined halogenated organic compound, vinylically-unsaturated precursor compound and organic hydrogen halide acceptor, including each of (a)-(ai);
(al) the diluent is an aqueous solution of up to 90 percent by volume of an organic solvent selected from nitriles, alcohols, linear or cyclic saturated esters, N,N-dialkylformamides, N-alkylpyrrolidinones, alkoxyalkanols, glycol ethers, ketones, dioxane, tetrahydrofuran, tetrahydropyran and hexaalkyl-phosphoramides, including each of (a)-(ai);
(am) the diluent is an aqueous solution of 10 to 90 percent by volume of N,N-dimethylformamide, including each of (a)-(ai);
(an) the diluent is an aqueous solution of 30 to 70 percent by volume of N-methylpyrrolidinone, including each of (a)-(ai);
(ao) the diluent is an aqueous solution of 30 to 70 percent by volume of N,N-dimethylformamide or N-methylpyrrolidinone and the diluent is present in an amount equal to at least 70 percent by weight of combined halogenated organic compound, vinylically-unsaturated precursor compound and organic hydrogen halide acceptor, including each of (a)-(ai);
(ap) a step of treating a resulting crude vinylically unsaturated product compound with a peroxide is included, including each of (a)-(ao);
(aq) the peroxide is aqueous hydrogen peroxide, including each of (a)-(ac);
(ar) the peroxide is tert-butyl hydroperoxide, including each of (a)-(ac);
(as) a step of chromatographing a resulting crude vinylically unsaturated product over silica or alumina is included, including each of (a)-(ar); and
(at) the further steps of treating a resulting crude vinylically unsaturated product with a peroxide and chromatographing the crude product over silica or alumina are included, including each of (a)-(ac).

Most preferred products, prepared by the process of this invention are those prepared from:
(a) 4-bromobenzocyclobutene and a mixture of m- and p-vinyltoluene;
(b) 4-bromobenzocyclobutene and 1,1'-divinyl-tetramethyldisiloxane;
(c) 4-bromobenzocyclobutene and divinylbenzene;
(d) ethylene and o-bromotoluene;
(e) ethylene and 4-bromobenzocyclobutene; and
(f) styrene and 4-bromobenzocyclobutene;

A most preferred catalyst complex is that formed from palladium (II) acetate and tris-(o-tolyl)phosphine.

A most preferred aqueous diluent is aqueous dimethylformamide, containing 30 to 70 percent by volume of N,N-dimethylformamide.

Most preferably, a resulting crude product is treated with aqueous hydrogen peroxide or with tert-butyl hydroperoxide.

In the following examples, the temperatures are set forth uncorrected in degrees Celsius. Unless otherwise indicated, all parts and percentages are by weight.

Samples are analyzed by GC using known standards of pure o- or p-vinyltoluene.

### Example 1 Coupling of o-Bromotoluene With Ethylene Using Triethylamine in Aqueous DMF

To the reactor is charged 374 g (2.187 mol) of 2-bromotoluene (98 percent, Aldrich), 442 g of triethylamine (reagent grade, Fisher), 0.59 g (0.0026 mol) of palladium (II) acetate (Engelhard), 3.18 g (0.0104 mol) of tris-(o-tolyl)phosphine (Strem Chemical Co.), 600 mL of N,N-dimethylformamide (Fisher) and 300 cc of deionized water. The reactor is sealed and attached to the reaction system. After stirring is initiated, the contents of the vessel are purged with nitrogen (5.44 atm, gauge; 0.55 MPag)) five times. The nitrogen is vented and the reactor is charged with ethylene (Scott Gas) to 5.10 atm, gauge (0.517 MPag) and held at this pressure.

A sample of about 5 mL is removed from the double-valved sample dip tube and used for a gas chromatography trace of the initial feed sample. Additional samples are removed during the reaction by discarding the first several samples to rinse the dip tube before using a later sample for GC analysis.

The temperature controller is set at 75°C and heating is initiated. The contents of the reactor reached the selected temperature after 30 minutes and is maintained at that temperature for the duration of a run.

A mixture of pure o-vinyltoluene and o-bromotoluene is analyzed on the GC. The contents of the reactor are sampled after 2, 4, 20.5, 28 and 45 hours. The conversion of o-bromotoluene is incomplete after 45 hours. The reactor is cooled to 30°C and opened in a hood for the addition of 0.030 g of palladium acetate and 1.59 g of tris-(o-tolyl)phosphine. The vessel is resealed, reattached to the system, purged with nitrogen as before and repressurized with 5.10 atm (gauge) (0.571 MPag) of ethylene. The contents of the reactor are heated to 75°C. At the end of 2 hours further heating, the sample removed shows complete conversion. The contents of reactor are stirred and cooled to room temperature. The reactor is vented and the reaction vessel is opened.

The contents of the reactor are poured into a 5-L three-necked glass round-bottom flask containing 1 L of methylene chloride (Burdick & Jackson) and 1 L of deionized water. The flask has a bottom dump valve and is stirred by an air-driven stirrer. The contents of the flask are stirred for 5 minutes and the top aqueous phase is discarded. The organic phase is washed with two 1.5-L portions of deionized water; two 250-mL portions of 5N HCl in 1250 mL of deionized water and two further 1.5-L portions of deionized water. The aqueous layer is discarded after each wash.

The organic phase is filtered through a short column prepared using a 300 mL Fisher brand microfiltration system with a 5-micrometer membrane filter, containing 150 mL of silica gel (Fisher, chromatographic 644 grade, type 150A, 100 to 200 mesh (150-75 µm)), topped with a 1.90 cm layer of magnesium sulfate. Filtration accomplishes removal of residual solids and catalyst and dries the solution.

Methylene chloride is removed from the filtrate on a Buchi Rotavaporizer Model RE 120 at 40°C at 15 mmHg (2kPa). The weight of crude reaction product is 227.6 g. The crude product is charged to a 500-mL round-bottom flask and attached to a 20.32-cm Bantamware column packed with glass helices, attached to a Bantamware one-piece distillation unit equipped with a vacuum-jacketed Vigreaux section and fraction cutter. The distillation setup is connected to a McCloud gauge, a nitrogen source, a dry ice (-78°C) trap, high-vacuum pump, heating mantle, temperature controller and temperature high-limit cutoff. The crude product is degassed and methylene chloride is removed with stirring until bubbling stopped. The distillation pot is heated to 50°C at about 0.4 mmHg (55 Pa). The first fraction (about 20 g) is collected at 24 to 25°C/0.35 mmHg (45 Pa). The second fraction (114.7 g) is collected at a pot temperature up to 55°C. The first and second fractions are identified as 100 percent pure o-vinyltoluene (GC). The dry ice trap contains 31.6 g of o-vinyltoluene, containing a trace of methylene chloride. The pot residue (53.2 g) crystallizes upon cooling. The pot residue contains 3.5 percent of o-vinyl-toluene (OVT), 60 percent of trans-disubstituted product, 30 percent of 1,1 (gem)--disubstituted product and 4 percent of tris-(o--tolyl)phosphine.

The combined yield of OVT (134.7 + 31.6) is 166.3 g (64 percent of theory).

### Example 2 Coupling of o-Bromotoluene With Ethylene Using Triethylamine in Aqueous DMF; Higher Catalyst Level.

The reaction is as in Example 1., except that 0.98 g (0.00437 mol) of palladium (II) acetate and 5.32 g of tris-(o-tolyl)phosphine are used as catalyst. The reactor is loaded as above and the temperature is increased to 120°C. Ethylene pressure is 13.6 atm, gauge (1.38 MPag). Samples are taken for analysis at 0, 3 and 5 h. At the end of 5 hours, only a trace of bromotoluene is present (GC). The product contains 75.3 percent of OVT, 16 percent of trans-diadduct and 6.8 percent of gem-diadduct. The reactor is shut down after 7 hours heating, as above.

The product is worked up as above to give 241 g of crude product. The mixture is purified by distillation at 8.0 mmHg (1.1 kPa), at which product boiled at 50°C to 51°C and no product was collected in the cold trap. The first cut gave 10 g of OVT (99 percent); the second cut gave 131 g of OVT (99.34 percent). A third cut (25.4 g 50°C to 51°C, 8 mmHg (1.1 kPa)) contains 98.6 percent of OVT and 1.2 percent of o-bromotoluene.

The pot residue (61.2 g) is mainly diadduct. The recovered OVT weighs 166.4 g (64.4 percent of theory).

### Example 3 Coupling of p-Bromotoluene With Ethylene Using Triethylamine in Aqueous DMF

The reaction is run as in Example 2, except that p-bromotoluene is used instead of o-bromotoluene. The reaction is run at 123°C, with cooling to 112°C after an initial exotherm. Samples are taken at 0, 3 and 5 hours and used for GC analyses. At the end of three hours, the GC shows 77.65 percent of p-vinyltoluene (PVT), less than 0.5 percent of p- bromotoluene, 18.1 percent of trans-diadduct and 3.05 of gem-diadduct. The reactor is cooled after 5 hours heating, at which point GC indicates complete conversion of bromotoluene to 73.5 percent PVT, 20.9 percent of trans-diadduct and 3.4 percent of gem-diadduct.

The product is worked up as in Example 2, except that 1.5 L of additional methylene chloride is used to dissolve precipitated solids. The solids are isolated, purified and characterized as trans-p-methylstilbene dicoupled product, m.p. 179°C (51 g). The total yield of isolated crude products is 225.5 g, comprising 72.9 percent of PVT, 22.0 percent of trans-diadduct, 3.2 percent of gem-diadduct and 1.2 percent of tris-(o-tolyl)phosphine.

### Example 4 Coupling of o-Bromotoluene With Ethylene Using Triethylamine in Aqueous DMF; Low Catalyst Level

The reaction is carried out as in Example 2, using a catalyst 0.098 g of palladium (II) acetate and 0.53 g of tris-(o-tolyl)phosphine. The contents of the reactor are sampled at 3, 5, 11, 13, 16, 22 and 25 hours. Conversions are 29, 42.1, 53, 56.8, 58.5, 59.2 and 59.2 percent, respectively. Yields of OVT (GC) are 26.4, 37.7, 46.7, 50, 51.3, 51.6 and 51.5 percent, respectively.

An additional charge of catalyst (0.098 g of palladium acetate, 0.53 g of tris-(o-tolyl)phosphine is added to the reaction mixture, which is heated to 120°C and repressurized with ethylene to 123.6 atm, gauge (12.52 MPag). The contents of the reactor are analyzed after 2, 18 and 21 hours further heating. The yield of OVT (GC) is 62.8, 78.9 and 78.0 percent, respectively. Conversion of o-bromotoluene is 72, 98.5 and 99.5 percent, respectively.

The washed, crude product weight 210.3 g. This polymerized partially. Monomer (10.3 g) is recovered by flash distillation

### Example 5 Coupling of p-Bromotoluene With Ethylene Using Potassium Acetate in Aqueous DMF

The reaction is run as in Example 3, except that 429 g (4.37 mol) of potassium acetate is used, instead of triethylamine. At the end of three hours, the reaction mixture contains 88.9 percent of p-vinyltoluene (PVT), 4.5 percent of bromotoluene, 3.76 percent of trans-diadduct and 2.26 percent of gemdiadduct. Attempted sampling of the contents of the reactor fails after 5 hours, because precipitated diadduct clogs the sample tube. The reaction is stopped after 7 hours. The yield of isolated crude products is 225.3 g, comprising 82.8 percent of p-vinyltoluene, 3.86 percent of trans-diadduct, 8.4 percent of gem-diadduct and 3.21 percent of tris-(o-tolyl)phosphine.

### Example 6 Coupling of o-Bromotoluene With Ethylene Using Triethylamine in Aqueous DMF; Low Temperature; High Pressure

The reaction is run as in Example 2, using a reactor temperature of 75°C and ethylene pressure of 13.6 atm, gauge (1.38 MPag). The contents of the reactor are sampled at 3, 5, 7.25, 9, 10, 14, 16, 18 and 20 hours, at which times the content of OVT (GC) is 33, 60, 77, 84.4, 87.6, 90, 91, 91.2 and 91.2 percent, respectively. The 20-hour sample contains no detectable bromotoluene by capillary GC, of which the detection limit is 0.02 percent.

The rinsed, crude product weighs 239.5 g.

### Example 7

### Coupling of o-Bromotoluene With Ethylene Using Triethylamine in DMF; Comparative Example

An experiment is run in a 10-gallon (38 L) reactor, using the procedure from Example 1, except that no water is added. Build up of a salt cake on the stirrer impedes stirring. The salt cake must be removed after each run. Sampling of the reactor is complicated by accumulation of salts in the sampling tube.

### Example 8

### Coupling of 4-BrBCB With Ethylene Using Triethylamine in Aqueous DMF at 50°C

To a 300-mL Fluitron pressure reactor is charged 0.061 g of palladium acetate, 0.322 g of tris-(o-tolyl)phosphine, 50 g of 4-BrBCB, 55.2 g of triethylamine, 50 mL of N,N-dimethylformamide and 25 mL of water. The reactor is closed, purged with ethylene three times and filled and maintained with ethylene at 5.10 atm, gauge (0.517 MPag). The contents of the reactor are heated to 50°C. A sample after 20 hours heating shows incomplete conversion (GC). Heating is continued until 4-BrBCB conversion is nearly complete (140 h). The product contains 0.3 percent of BCB, 0.56 percent of BrBCB, 82.3 percent of 4-vinylBCB and 14.9 percent of 1,2-bis-(4-benzocyclobutenyl)ethylene.

### Example 9

### Coupling of 4-BrBCB With Ethylene Using Triethylamine in Aqueous DMF at 75°C

To the 2-L reactor, described in Example 1, is charged 1.27 g of palladium acetate, 10 g of tris-(o-tolyl)phosphine, 500 g of 4-BrBCB, 552 g of triethylamine, 1 g of MEHQ (hydroquinone monomethyl ether), 500 mL of N,N-dimethylformamide and 250 mL of deionized water. The reactor is closed and the reaction is run at 75°C under 20.40 atm (gauge) (2.067 MPag) of ethylene pressure. At the end of 24 hours heating, a sample contained 86.68 percent of vinylbenzocyclobutene, 2.32 percent of 4-BrBCB and 7.88 percent of 1,2-bis-(4-benzocyclobutenyl)ethylene.

The mixture is removed from the reactor. The organic phase is washed with water, neutralized with aqueous HCI and cooled to precipitate bis-adduct. The crude product weighs 359 g (theoretical 355 g). The product is distilled in vacuo.

### Example 10

### Coupling of 4-BrBCB With Ethylene Using Triethylamine in DMF; Comparative Example

The reaction is run as in Example 9, using 500 g of 4-BrBCB, 552 g of triethylamine, 1.9 g of palladium (II) acetate, 1.0 g of tris-(o-tolyl)phosphine, 1.0 g of MEHQ and 750 mL of N,N-dimethylformamide instead of the aqueous mixture. The reaction mixture is heated to 85°C under 20.40 atm (gauge) (2.067 MPag) of ethylene. After 24 hours heating, the reaction mixtures contains 1.29 percent of 4-BrBCB, 87.5 percent of vinyl BCB and 5.87 percent of bis-BCB-ethylene.

An accumulation of solids at the top of the reactor seals the top of the reactor to the body of the reactor. A screwdriver is required to pry the top of the reactor from the body. A donut-shaped salt cake lines the top and bottom of the reactor. The salt cake is chipped off and ground up. The salt cake is soluble in water. The salt cake appears to be mainly triethylamine hydrobromide.

The weight of crude product, worked up as above, is 304 g (86 percent).

### Example 11

### Coupling of 4-BrBCB With Ethylene Using Triethylamine in DMF; Comparative Example

The reaction is run as in Example 10, the temperature being maintained at about 85°C and ethylene pressure being maintained at about 6.80 atm (gauge) (0.689 MPag). The solids content of the resulting mixture is very high. Addition of water to the mixture causes solids to float on the mixture.

### Example 12

### Coupling of 4-BrBCB With Ethylene Using Triethylamine in Aqueous DMF to Make Trans-1,2-Bis-(4-benzocyclobutenyl)-ethylene

The same reactor, as in Example 1, is charged 0.28 g of palladium acetate, 1.50 g of tris-(o-tolyl)-phosphine, 150 g of 4-BrBCB, 173 g of triethylamine, 350 mL of N,N-dimethylformamide and 175 mL of deionized water. The reactor is closed and purged three times with ethylene (5.10 atm, gauge; 0.517 MPag) and maintained at this pressure. The reactor is heated and maintained at 72°C to 76°C.

After four hours heating a sample contains 44.9 percent of vinyl BCB and 52.4 percent of unreacted 4-BrBCB. Ethylene is vented from the reactor and replaced with nitrogen (5.44 atm, gauge; 0.551 MPag). Heating is continued. At the end of 49.5 hours overall heating, a sample contains (GC) 90.0 percent of trans-1,2-bis-(4--benzocyclobutenyl)ethylene, 3.15 percent of vinyl BCB and 3.0 percent of BrBCB.

The reactor is opened and the contents are poured into a 5-L round-bottom flask equipped with a stirring assembly and a bottom take-off. Deionized water is added. The mixture is stirred and a resulting aqueous phase is separated and removed. Toluene (200 mL) is added to the mixture to dissolve precipitated solids and the organic layer is washed with two 1.5-L portions of deionized water. The organic phase is washed with 250 mL of 2N HCl in 1250 mL of deionized water to a final pH of about 3.

The organic layer is separated and passed through a layer of silica gel (5.08 cm), topped by a layer (1.27 cm) of magnesium sulfate, supported on a 1.0 micrometer membrane filter.

Toluene is removed from the filtrate under vacuum to leave 102 g of crude product (about 88 percent of theory) in the form of a slurry.

Product recrystallized from 100 mL of ethyl acetate gives 83.0 g of trans-1,2-bis-(4-benzocyclobutenyl)ethylene, m.p. 132°C. GC analysis detects one peak (99.7 area percent).

### Example 13

### Coupling of 4-BrBCB With Ethylene Using Triethylamine in Aqueous DMF to Make 1,1,2-Tris-(4-benzocyclobutenyl)ethylene

To a 100-mL one-necked flask equipped with a polytetrafluoroethylene-coated magnetic stirring bar and thermometer well are charged 12.5 mL of deionized water, 5.73 g (0.0568 mol) of triethylamine, 0.012 g (0.000026 mol) of palladium (II) acetate, 0.062 g (0.000103 mol) of tris-(o-tolyl)phosphine, 6.00 g (0.0258 mol) of trans-1,2-bis-(4-benzocyclobutenyl)ethylene and 25 mL of N,N-dimethylformamide.

The resulting solution is purged with a stream of nitrogen for 30 minutes, after which the solution is heated to 90°C. The reaction mixture is heated and stirred at 90°C for 115 hours. Gas chromatographic analysis of the crude reaction mixture indicates the mixture contains 0.5 percent of 4-BrBCB, 0.4 percent of trans-1,2-bis-(4-benzocyclobutenyl)ethylene and 88.0 percent of 1,1,2-tris-(4-benzocyclobutenyl)ethylene.

The reaction mixture is allowed to cool to 80°C and diluted with 40 mL of toluene. The organic layer is washed with 50 mL portions of 0.1 N HCl until the aqueous layer remains acidic. The organic layer is washed with two 50-mL portions of deionized water and dried over magnesium sulfate. The solvent is removed in vacuo to give 8.3 g of a yellow oil.

The oil is dissolved in heptane and cooled to 5°C. The resulting crystals are separated by filtration to give a 5.6 g of 1,1,2-tris-(4-benzocyclobutenyl)-ethylene, m.p. 87°C to 89°C.

¹H NMR (CDCl₃); δ 6.6-7.3 (m, 10 H), 2.9-3.3 (d, 12 H); ¹³C[¹H] NMR (CD₂Cl₂, 75.1 MHz) 146.74, 146.31, 145.97, 145.82, 145.56, 145.09, 144.16, 144.00, 140.47, 137.33, 129.59, 129.50, 128.76, 127.22, 124.98, 123.81, 123.21, 122.67, 122.62, 122.39, 29.80, 29.59.

### Example 14

### Coupling of 4-BrBCB With Ethylene Using Potassium Acetate in Aqueous DMF to Make trans-1,2-Bis- (4-benzocyclobutenyl)-ethylene

An experiment is run as in Example 12, using 0.28 g of palladium acetate, 1.50 g of tris-(o-tolyl)-phosphine, 150 g of 4-BrBCB, 350 mL of N,N-dimethylformamide, 175 mL of deionized water and potassium acetate as hydrogen halide acceptor.

Product recrystallized from 100 mL of ethyl acetate is identified as trans-1,2-bis-(4-benzocyclobutenyl)-ethylene, m.p. 132°C. GC analysis detects one peak (99.7 area percent).

### Example 15

### Coupling of 4-BrBCB With Bis-BCB-ethylene Using Potassium Acetate in Aqueous DMF to Make 1,1,2- Tris-BCBethylene

To a 100-mL one-necked flask equipped with a polytetrafluoroethylene-coated magnetic stirring bar and thermometer well as charged 12.5 mL of deionized water, 5.57 g (0.0568 mol) of potassium acetate, 0.012 g (0.000026 mol) of palladium (II) acetate, 0.062 g (0.000103 mol) of tris-(o-tolyl)phosphine, 6.00 g (0.0258 mol) of trans-1,2-bis-(4-benzocyclobutenyl)-ethylene, 10.0 g of 4-BrBCB and 25 mL of N,N-dimethylformamide.

The reaction is run as in the foregoing examples to give 1,1,2-tris-BCB-ethylene, m.p. 87-89°C.

¹H NMR (CDCl₃); δ 6.6-7.3 (m, 10 H), 2.9-3.3 (d, 12 H); ¹³C[¹H] NMR (CD₂Cl₂, 75.1 MHz) 146.74, 146.31, 145.97, 145.82, 145.56, 145.09, 144.16, 144.00, 140.47, 137.33, 129.59, 129.50, 128.76, 127.22, 124.98, 123.81, 123.21, 122.67, 122.62, 122.39, 29.80, 29.59.

### Example 16

### Coupling of 4-BrBCB with 1,1'-Divinylbenzene Using Triethylamine in Water

The following reactants are charged to a 100-mL round bottom flask, fitted out with a reflux condenser and a

| | |
|---|---|
| 10 g | 4-BrBCB (Dow) |
| 3.64 g | divinylbenzene (95 percent - m/p 2/1, Dow) |
| 11 g | triethylamine (Fisher, reagent grade) |
| 37.5 g | deionized water |
| 0.0122 g | palladium (II) acetate (Engelhard) |
| 0.0664 | tris-(o-tolyl)phosphine (Strem Chemical Co.) |

The resulting two-phase liquid system is purged with nitrogen and stirred and heated under reflux (about 90°C). The reaction mixture turns black soon after the reflux temperature is reached. After 24 hours heating, the mixture is cooled and each layer of the resulting mixture is analyzed by GC and LC (Hewlett-Packard 5710GC, as above, and HPLC, as above). Conversion to product, which is in the upper layer, is complete.

Some solid precipitates from the cooled mixture. Toluene (25 mL) is added to the mixture, which is heated and poured while hot into a 250-mL separatory funnel. The bottom aqueous layer is removed. The organic layer is diluted with 25 mL of toluene and 100 mL of water and washed with four 100-mL portions of water.

The washed organic layer is heated to dissolve some solids and passed through a membrane filter (0.5 micrometer) to remove residual palladium.

The filtered solution is concentrated using a rotary evaporator to produce 9.6 g (52 percent of theory) of crystalline divinylbenzene-bis-BCB, alternatively, bis-[(4-benzocyclobutenyl)vinyl]benzene.

Analyses: Reversed-phase HPLC, as above. M.p. 148°C to 152°C, (Fisher-Johns melting point apparatus).

### Example 17

### Coupling of 4-BrBCB with m/p-Divinylbenzene Using Triethylamine in Aqueous DMF

To a 5-L, round-bottom flask, fitted out with a heating mantle, bottom dump valve, thermowell, stirrer assembly and reflux condenser, is charged 0.62 g of palladium acetate, 3.35 g of tris-(o-tolyl)phosphine, 500 g of 4-BrBCB, 551.5 g of triethyl-amine, 712.5 g of fresh commercial 80 percent m/p-divinylbenzene, 1000 mL of N,N-dimethylformamide and 500 mL of deionized water.

The reactor is degassed with nitrogen and heated to 92°C to 94°C. At the end of 5 hours heating, a sample contains 0.06 percent of 4-BrBCB. The reactor is held at temperature for 23 hours, at the end of which polymer buildup on the reactor walls, is observed. A sample of the liquid phase contains 0.05 percent of BrBCB, 49.16 percent of divinylbenzenes (DVB) and 41.4 percent of trans-bis-[(4-benzo-cyclobutenyl)vinyl]-benzene (trans-DVB-BCB).

The reactor is cooled and 2 L of toluene are added. The water layer, which separates, is discarded. The organic layer is washed with three 1.5-L portions of deionized water with 200 mL of 5N HCl in 1300 mL of deionized water and with two further 1.5-L portions of deionized water.

The organic phase is filtered through 150 mL of silica, topped with 40 g of magnesium sulfate, on a 5-micrometer membrane filter. Toluene is stripped from the filtrate at 70°C under full vacuum to yield 226.8 g of crude product. After two days standing in a refrigerator, 25.5 g of solid material is removed by filtration. The filtrate contains 59 percent of DVB and 37 percent of DVB-BCB. The solid contains 9.6 percent of DVB, 66 percent of DVB-BCB and 21 percent of bis-[(4-benzocyclobutenyl)vinyl]benzene (DVB-BCB₂) (LC). The liquid is distilled in a short path molecular distillation apparatus at 135°C/l micrometer. The overheads fraction (66.85 g) contains 93.26 percent of DVB-BCB mixed isomers and 5.86 percent of DVB (LC).

### Example 18

### Coupling of 4-BrBCB with Mixed Isomers of Vinyltoluene in Aqueous Dimethylformamide

The following materials are charged to a 5-L round-bottom flask, fitted out with a reflux condenser and magnetic stirring bar:

| | |
|---|---|
| 800 g | 4-BrBCB |
| 516 g | vinyltoluene (m/p = 70/30) |
| 968 g | triethylamine (Fisher, reagent grade) |
| 1000 mL | N,N-dimethylformamide (DMF, Fisher, certified A.C.S.) |
| 500 mL | deionized water |
| 0.976 g | palladium (II) acetate (Engelhard) |
| 5.344 g | tris-(o-tolyl)phosphine (Strem Chemicals, Inc.) |

The resulting two-phase liquid mixture is purged with nitrogen and then stirred and heated under reflux (about 90°C) under a stream of nitrogen. The reaction mixture remains essentially clear until all of the 4-BrBCB is reacted. At this point, a dark precipitate, which concentrates in the upper layer, is formed. The reaction is complete in 12 to 16 hours.

At the end of the reaction, the mixture is cooled to room temperature and the clear bottom layer is removed by decantation in a separatory funnel. About 1500 mL of toluene is added to the upper layer and about 1000 mL of deionized water is added. The mixture is shaken and the lower aqueous layer is removed by decantation, as before. The toluene layer is extracted with 1000 mL of fresh deionized water.

The resulting toluene solution is split into two portions. Each half is passed through a column (3.175 cm inner diameter by 66.04 cm high) packed with alumina (Fisher 80 to 200 mesh (180-75 µm), basic alumina, Brockman Activity 1) to remove particulates. The columns are prepared by prepacking columns with 500 g of alumina, in the form of a slurry in toluene. After loading the column, the column is eluted with toluene. The effluent is a clear golden yellow solution, which is assayed for residual tris-(o-tolyl)phosphine (as phosphorus) by neutron activation analysis. Tri-(o-tolyl)phosphine is removed by stirring the toluene solution overnight with 250 mL of deionized water and 20 mL of 30 percent hydrogen peroxide at room temperature.

The lower toluene layer of the resulting two-phase solution is assayed by gas chromatography for the presence of unreacted tris-(o-tolyl)phosphine.

After all of the residual triarylphosphine has been converted to the phosphine oxide, the toluene solution is passed through a column packed with alumina, as above, to remove additional particulate matter and phosphine oxide. The effluent from the column is reduced in volume by removing toluene using a rotating evaporator. The product crystallizes after about 2 days at room temperature.

The isolated yield is 812 g (85 percent of theory).

The product is identical to a known sample, compared by gas chromatography, GC/MS and proton NMR.

The product contains less than: 0.5 ppm Pd, 5 ppm P (not detectable by gas chromatography), 1 ppm Cl, 0.5 ppm Br, or 1 ppm Na by x-ray fluorescence or neutron activation analyses.

### Example 19

### Coupling of 4-BrBCB with m/p-Vinyltoluene Using Triethylamine in Water

To a 100-mL round-bottom flask, fitted out with a reflux condenser and magnetic stirring bar, is charged:

| | |
|---|---|
| 10 g | 4-BrBCB |
| 6.44 g | vinyltoluene (m/p = 70/30, Dow Chemical Co.) llgtriethylamine (Fisher, reagent grade) |
| 37.5 mL | deionized water |
| 0.0122 g | palladium (II) acetate (Engelhard) |
| 0.0664 g | tris-(o-tolyl)phosphine (Strem Chemical Co.) |

The resulting two-phase liquid mixture is purged with nitrogen and stirred and heated under reflux (about 90°C). The reaction mixture turns black at the end of the reaction. At the end of 24 hours, the upper layer contains all the reactants and the lower layer contains no organic materials, detectable by capillary GC. These analytical data are consistent with complete reaction of 4-BrBCB.

At the end of 24 hours, the reaction mixture is cooled to room temperature and diluted with 25 mL of toluene. The resulting mixture is transferred to a 250-mL separatory funnel. The bottom aqueous layer is removed and the upper organic layer is diluted with 25 mL of toluene. The resulting organic layer is washed with four 100-mL portions of deionized water and filtered through magnesium sulfate to remove residual water and palladium.

The organic filtrate is charged to a 100-mL round bottom flask and stirred with 10 mL of 30 percent hydrogen peroxide for 4 hours at room temperature. The layers are separated and the crude organic layer is washed with three 100-mL portions of distilled water and filtered through magnesium sulfate. The filtrate is passed over a column (1.91 cm inner diameter, 19.05 cm in length), packed with basic alumina (about 15 g, Fisher 80 to 200 mesh (180-75 µm), basic alumina, Brockman Activity 1). Toluene is removed from the column effluent using a rotary evaporator. The liquid product contains m/p-[(4-benzocyclobutenyl)vinyl] toluene (GC, as above) which crystallizes after standing overnight at room temperature to give 11 g of product (92 percent of theory).

The product contains: less than 1 ppm Pd, about 4 ppm P, about 2 ppm Cl, about 1 ppm Br and less than 1 ppm Na by x-ray fluorescence or neutron activation analyses.

### Example 20

### Coupling of 4-BrBCB With m/p-Vinyltoluene Using Triethylamine in Water

The following reactants are charged to a 100-mL round-bottom flask, fitted out with a reflux condenser and magnetic stirring bar:

| | |
|---|---|
| 10 g | 4-BrBCB (0.054 mol, Dow) |
| 6.44 g | vinyltoluene (m/p = 70/30, 0.054 mol, Dow) |
| 5.51 g | triethylamine (0.054 mol, Fisher, reagent grade) |
| 37.5 mL | deionized water |
| 0.0122 g | palladium (II) acetate (Engelhard) |
| 0.0664 g | tris-(o-tolyl)phosphine (Strem Chemical Co.) |

The reaction is carried out as in Example 1. At the end of 22 hours, the reaction mixture is analyzed by GC and contains about 5 percent of unreacted BrBCB and vinyltoluene and a trace of triethylamine. The major product is vinyltoluene adducts of BCB, systematically named 3-[2-(3-or 4-methylphenyl)-ethenyl]bicyclo[4.2.0]-octa-1,3,5-triene.

The product forms a glob in the reactor and is difficult to stir.

This example shows that using an excess of triethylamine acid acceptor is not required.

### Example 21

### Coupling of 4-BrBCB with m/p-Vinyltoluene Using Picoline in Aqueous DMF; Comparative Example

To a 100-mL flask, equipped with a magnetic stirring bar, thermowell and condenser, are charged 0.0122 g of palladium acetate, 0.0664 g of tris-(o-tolyl)phosphine, 10.0 g of 4-BrBCB, 6.44 g of commercial grade m/p-vinyltoluene, 10.17 g of 2-picoline, 25 mL of N,N-dimethylformamide and 12.5 g of HPLC grade water. The system is purged with nitrogen and then heated to about 95°C for 24 hours. GC analysis shows only a trace of addition product.

### Example 22

### Coupling Using Differing Phosphines

(a) Experiments are run as in Example 19, using triphenylphosphine instead of tris-(o-tolyl)phosphine. Similar results are obtained. The catalytic species is believed to be bis(triphenylphosphine)-palladium (O).
(b) Similar results are obtained, using tributylphosphine as a catalyst component.
(c) Similar results are obtained using as a catalyst component tris-(dibenzylideneacetone)di- palladium (O) and triphenylphosphine or dichloro(triphenylphosphine)palladium (II).

### Example 23

### Coupling of 4-BrBCB with Vinyltoluene Using NaHCO₃ in Aqueous DMF

To a 100-mL flask, equipped with a magnetic stirring bar, thermowell and condenser, are charged 0.0122 g of palladium acetate, 0.0664 g of tris-(o tolyl)phosphine, 10.0 g of 4-BrBCB, 6.44 g of commercial grade m/p-vinyltoluene, 9.17 g of sodium hydrogen carbonate, 25 mL of N,N-dimethylformamide and 12.5 g of HPLC grade water. The system is purged with nitrogen and then heated to about 95°C for 17 hours. Some of the sodium hydrogen carbonate remained as a solid at the bottom of the reaction mixture. After 17 hours, a sample of the top organic layer was analyzed by GC. The sample contains no Br-BCB, a trace of vinyltoluenes and 1-(4-benzocyclobutenyl)-2-(m/p-tolyl)-ethylene.

The contents of the flask are cooled and added to a mixture of 100 mL of deionized water and 25 mL of toluene. The organic phase is washed with four 100 mL portions of deionized water to remove sodium bromide and unreacted sodium hydrogen carbonate. After separating the organic layer, which is dried over magnesium sulfate, toluene in the organic layer is removed on a rotavaporator. The product is analyzed by GC/MS and has a parent peak of 220 mass units.

### Example 24

### Coupling of 4-BrBCB with Vinyltoluene using 2-Picoline and KOH in Aqueous DMF

The reaction is run as in Example 23, except that 10.17 g of 2-picoline is used as hydrogen halide acceptor. After 24 hours heating at 95°C, only a trace of product is obtained (GC). Potassium hydroxide (one equivalent) is added to the mixture, which is heated at 95°C overnight. The resulting mixture contains less than 1 percent of 4-BrBCB; the balance is 1-(4-benzocyclobutenyl)-2-(tolyl)ethylene.

### Example 25

### Coupling of 4-BrBCB with Vinyltoluene NaOH in Aqueous DMF; Comparative Example Without Phosphine

To a 100-mL round-bottom flask, equipped with a stirring bar, reflux condenser and thermowell, is charged 10.0 g of 4-BrBCB, 6.44 g of commercial m/p-vinyltoluene, 2.19 g of sodium hydroxide, 0.0122 g of palladium acetate, 225 mL of N,N-dimethyl-formamide and 12.5 mL of water. The reaction mixture is heated at 95°C for 20 hours. A sample contains (GC) 30 percent of Br-BCB and 33 percent of 1-(4-benzocyclobutenyl)-2-(tolyl)-ethylene. After adding 0.0122 g of palladium acetate, the mixture is heated at 95°C for two days more. The yield of 1-(4-benzocyclobutenyl)-2-(tolyl)-ethylene increases to 35 percent (GC).

### Example 26

### Coupling of Bromobenzene with Ethyl Acrylate Using Triethylamine in Aqueous DMF

To a 100-mL one-necked flask, fitted out with a polytetrafluoroethylene-covered magnetic stirring bar, reflux condenser and thermometer well, is charged 8.57 g (0.0546 mol) of bromobenzene, 5.46 g (0.0546 mol) of ethyl acrylate, 11.02 g (0.1092 mol) of triethylamine, 0.0246 g (0.00011 mol) of palladium (II) acetate, 0.134 g (0.00044 mol) of tris-(o-tolyl)phosphine, 25 mL of N,N-dimethylformamide and 12.5 mL of deionized water. The atmosphere over the solution is purged with nitrogen and a nitrogen pad is maintained over the mixture during the reaction. The reaction mixture is heated to 90°C for 16 hours. Gas chromatographic analysis of the mixture shows that the reaction mixture contains 94.7 percent of ethyl 3-phenyl-propenoate.

¹H NMR (CDCl₃): δ 7.6 (d, 1H, J= 32 Hz); 6.4- 7.4 (m, 5H), 6.3 (d, 1H, J = 32 Hz), 4.2 (q, 2H, J = 14 Hz), 1.3 (t, 3H, J = 14 Hz).

### Example 27

### Coupling of 4-BrBCB and Ethyl Acrylate Using Potassium Acetate in Aqueous DMF

To a 5-L three-necked flask, equipped with an air-driven stirrer, condenser and thermocouple well, is charged 2.02 g of palladium acetate, 10.96 g of tris-(o-tolyl)-phosphine, 502.3 g of 4-BrBCB, 276 g of ethyl acrylate, 542 g of potassium acetate, 823 mL of N,N-dimethylformamide and 411 mL of deionized water. The reactor is purged with nitrogen and a nitrogen atmosphere is maintained over the reactor. The reaction mixture is stirred and heated to 93°C for 16 hours. A sample of the product at this point contains no BrBCB and 84.8 percent of trans-BCB-CH=CHCOOC₂H₅.

The reactor is cooled and 1 L of deionized water is added. The water layer is separated and discarded. The mixture is diluted with 1 L of toluene and the organic phase is washed with five 1-L portions of deionized water. The organic phase is filtered through 150 mL of silica gel, topped by 40 g of magnesium sulfate, on a membrane filter. Solvent is evaporated from the filtrate to give 521 g of crude product.

The proton NMR spectrum of the crude product is compared to that of ethyl cinnamate. Part of the crude product is purified by vacuum distillation at 103°C/0.24 mmHg to purity above 98 percent (GC). This material has a parent ion at m/e = 202 by GC/MS.

### Example 28

### Coupling of 4-BrBCB and Styrene Using Potassium Acetate in Aqueous DMF

To a 5-L round-bottom flask, equipped with a heating mantle, bottom dump valve, thermowell, reflux condenser and stirrer assembly, is charged 0.98 g of palladium acetate, 5.327 g of tris-(o-tolyl)phosphine, 800 g of 4-BrBCB, 1072.78 g of potassium acetate, 1000 mL of N,N-dimethylformamide, 500 mL of deionized water and 454.40 g of freshly-distilled styrene. The reactor is degassed with nitrogen, heated to about 93°C and maintained at that temperature for 27 hours. The crude reaction product contains 0.85 percent of BrBCB and 96.74 percent of styrylbenzocyclobutene.

The reaction mixture is cooled to 70°C and 1 L of hot deionized water and 1 L of toluene are added. The resulting water layer is separated and discarded. The organic layer is washed with three further 1.5-L portions of deionized water. The organic phase is cooled to room temperature and diluted with 500 mL of toluene. The resulting organic phase is filtered through 150 g of silica gel, topped with 40 g of magnesium sulfate, on a 1.0 micrometer membrane filter. Toluene is removed from the filtrate on a rotavap to leave 975.45 g of crude product. This is recrystallized from 2 L of hot ethyl acetate. The crystallized product is filtered, washed with ethanol and dried overnight at 50°C. The first crop weigh 260 g, the second 378 g and the third 71.2 g. The product corresponds to a fully characterized standard.

### Example 29

### Coupling of 4-BrBCB With 4-Acetoxystyrene Using Potassium Acetate in Aqueous DMF

To a 5-L round-bottom flask, equipped with heating mantle, bottom dump valve, thermowell, reflux condenser and stirrer assembly, is charged 0.604 g of palladium acetate, 3.374 g of tris-(o-tolyl)phosphine,480.91 g of 4-BrBCB, 515.31 g of potassium acetate in 575 mL of deionized water, 1150 mL of N,N-dimethylformamide and 341.3 g of 4-acetoxystyrene.

The reactor is degassed with nitrogen and heated to about 90°C and held at about 90°C for 15.5 h. The crude reaction product (GC) contains 13 percent of 4-BrBCB, 37 percent of [(4-benzocyclobutenyl) vinyl]phenol and 33 percent of [(4-benzocyclobutenyl) vinyl]phenyl acetate. A sample, taken after 19.5 hours heating contains 9.6 percent of 4-BrBCB, 44 percent of [4-benzocyclobutenyl)vinyl]phenol and 25 percent of [(4-benzocyclobutenyl)vinyl]phenyl acetate.

The crude product is treated with caustic to form a water-soluble sodium phenate, which is acidified and separated to yield 350 g of high purity [(4-cyclobutenyl)vinyl]phenol (97 percent by GC area).

### Example 30

### Coupling of 4-BrBCB with DVS Using Triethylamine in Aqueous DMF.

The following reagents are charged to a 100-mL round-bottom flask, fitted out with a reflux condenser and magnetic stirring bar:

| | |
|---|---|
| 10 g | 4-BrBCB |
| 5.08 | DVS (Dow Corning) |
| 11.05 g | triethylamine (Fisher, reagent grade) |
| 25 mL | N,N-dimethylformamide (DMF, Fisher, certified A.C.S. grade) |
| 12.5 mL | deionized water |
| 0.026 g | palladium (II) acetate (Engelhard) |
| 0.146 g | tris-(o-tolyl)phosphine (Strem Chemicals, Inc.) |

The resulting two-phase liquid mixture is purged with nitrogen and stirred and heated under reflux (about 90°C) under a stream of nitrogen. The upper layer remains clear until all of the BrBCB has reacted. At this point, a dark precipitate, which is concentrated in the upper layer, is formed. The reaction is complete, as indicated by metal deposition, after 12 to 16 hours.

The crude reaction mixture is cooled, poured into 150 mL of deionized water and extracted with two 40-mL portions of pure n-heptane. The combined heptane extracts are washed with three 150-mL portions of deionized water.

The crude product is analyzed by gas chromatography and liquid chromatography. The product contains no 4-BrBCB or divinyltetramethyldisiloxane.

The product-containing heptane extract is charged to a 100-mL round-bottom flask with 15 mL of methanol and 10 mL of 30 percent hydrogen peroxide solution and stirred overnight. The resulting solution is filtered through a 0.5 micrometer membrane filter and rinsed with two 150-mL portions of deionized water. A white solid, which precipitates and which is removed by filtration, is identified as bis-BCB-ethylene, in an amount of about 0.2 g.

Heptane is removed from the resulting clear solution by evaporation using a rotary evaporator 8.6 g of crude yellow product (40 percent of theory).

The crude product is diluted with 15 mL of n-heptane and passed through a column (1.91 cm inner diameter, 22.85 cm in length), packed with 25 g of J. T. Baker chromatography grade silica gel (60 to 230 Å; 6-23 nm). The material on the column is eluted with 100 mL of heptane to give 7.0 g of product. A dark band remains at about 3.81 cm from the top of the column and a light yellow band about 12.7 cm from the top of the column. Product is obtained by removing heptane from the main product fraction using a rotary evaporator and distilling the residue in a wiped film still.

The resulting product contains: less than 5 ppm Pd, less than 5 ppm P, about 4 ppm Cl, less than 1 ppm Br and less than 1 ppm Na by x-ray fluorescence or neutron activation analyses.

### Example 31

### Coupling of 4-BrBCB with DVS Using Triethylamine in Water

To a 100-mL round-bottom flask fitted with reflux condenser and magnetic stirring bar is charged:

| | |
|---|---|
| 10 g | 4-BrBCB |
| 5.08 g | DVS (Dow Corning) |
| 11 g | triethylamine (Fisher, reagent grade) |
| 37.5 mL | deionized water |
| 0.026 g | palladium (II) acetate (Engelhard) |
| 0.146 g | tris-(o-tolyl)phosphine (Strem Chemical Co.) |

The two-phase reaction mixture is purged with nitrogen and stirred and heated under reflux. The reaction mixture turns black shortly after reflux temperature (about 90°C) is reached. The reaction is continued for 28 hours, at the end of which the upper layer contains both reactants and products, detectable by GC.

At the end of 28 hours, the GC analysis shows that the conversion of BrBCB is incomplete. Stirring and heating under reflux is continued for a total of 117 hours, after which the reaction mixture is cooled and poured into a 150-mL separatory funnel and diluted with 40 mL of n-heptane. The aqueous layer is removed and the organic layer is washed with four 75-mL portions of distilled water. The resulting light yellow organic layer is dried by being passed over a layer of magnesium sulfate on a medium glass fritted filter.

The resulting organic filtrate is transferred to a 100-mL round bottom flask and stirred for 20 hours at room temperature with 15 mL of 30 percent hydrogen peroxide. The liquid layers are separated and the organic layer is transferred to a separatory funnel and washed with four 65-mL portions of distilled water. The washed organic layer is filtered through a layer of magnesium sulfate on a fritted glass filtration funnel. The filtrate is concentrated using a rotary evaporator to yield 9.3 g (44 percent of theory) of crude DVS-BCB₂, of which the major component is formally known as 1,3-bis(2-bicyclo[4.2.0]-octa-1,3,5-trien-3-ylethenyl)-1,1,3,3-tetramethyldi-siloxane.

The crude product is taken up in about 20 mL of n-heptane and passed through a column (1.905 cm inner diameter, 33.02 cm in length) column, packed with 40 g of J. T. Baker chromatography grade silica gel (60 to 230Å; 6-23 nm). The material on the column is eluted with 600 mL of n-heptane to give 6.7 g of pure product (more than 90 percent of DVS-BCB₂ isomers) after removing n-heptane on a rotary evaporator. An additional 1.5 g of yellow product was eluted from the column with acetone. This is lower purity product.

### Example 32

### Coupling of 4-BrBCB with DVS Using Triethylamine in Aqueous DMF

To a 250 mL-round bottom flask, equipped with magnetic stirrer and reflux condenser, are charged 10.0 g of 4-BrBCB, 5.08 g of DVS, 11.0 g of triethylamine, 0.0246 g of palladium (II) acetate, 0.134 g of tris-(o-tolyl)phosphine, 25 mL of deionized water and 50 mL of DMF. The reactor is purged with nitrogen for 30 minutes at room temperature and then heated under reflux for 20 hours. GC Analysis shows that all of the BrBCB is reacted.

At the end of 24 hours under reflux, the reaction mixture contains 5.3 percent of 4-vinylbenzocyclobutene, 9.6 percent of DVS-BCB, 17.4 percent of bis-BCB-ethylene and 48.1 percent of DVS- -BCB₂.

This experiment shows that increasing the amount of mixed solvent has little effect on the reaction.

### Example 33

### Coupling of 4-BrBCB and DVS Using Triethylamine and Potassium Acetate in Aqueous DMF

A 22-L reactor is prepared for the reaction by turning on nitrogen, introduced through a bubbler; turning on water for a reflux condenser; checking that dump valves at the bottom of the reactor are closed; charging reagents to the reactor; turning on the agitator. Nitrogen is passed through the liquid charge for 30 minutes. The heating cycle is initiated at this point and the temperature is checked after 45 minutes to 1 hour.

A typical reactor charge is:

| | |
|---|---|
| 3200 g | 4-BrBCB |
| 1626 g | DVS (Dow Corning X1-2297 fluid) |
| 1755 g | triethylamine (Fisher Scientific, Reagent Grade, 99 percent) |
| 43 g | tris-(o-tolyl)phosphine (Strem Chemical Co.) |
| 8 g | palladium (II) acetate (Engelhard, 47.4 percent Pd) |
| 2557 g | potassium acetate (Fisher Scientific, certified ACS) |
| 4800 mL | DMF (4000 mL to reactor; 800 mL to rinse in catalyst mixture, Fisher Scientific, ACS) |
| 2400 mL | deionized water |

The reactor temperature is controlled to 94°C to 97°C for the duration of heating under reflux. At the end of 24 hours, the reaction mixture is checked by GC for unreacted BrBCB, using a capillary GC 5890 apparatus.

If the BrBCB content is above about 2 percent, the reaction is continued and samples are taken every few hours and conversion of BrBCB is rechecked. If more than 2 percent of BrBCB is unreacted after 48 hours, an additional charge of catalyst is employed.

When 98 percent of the BrBCB is reacted, heating for the reactor is shut off and the contents of the reactor are allowed to cool to 70°C over about 1.5 hours. Part of the water (ca 9.5 L) is removed through a bottom dump. To the reactor is added 3 L of heptane and 10 L of deionized water and the contents of the reactor are stirred for 15 minutes and allowed to settle. Part of the water layer is removed through a bottom dump.

An additional 10 L of deionized water is added and the contents of the reactor are stirred for 15 minutes and allowed to settle. Part of the water layer is removed, as above.

To the reactor are added 10 L of deionized water and 500 mL of 5N hydrochloric acid. The resulting mixture is stirred for 15 minutes and allowed to settle. Part of the water layer is removed, as above.

Deionized water (10 L) is added and the mixture is stirred for 15 minutes and allowed to settle. The pH or the mixture is checked. Part of the water is removed.

Another portion is deionized water (10 L) is added. The mixture is stirred for 10 minutes and allowed to settle. The pH is checked. Part of the water layer is removed. If pH is more acidic than 6 to 7, the contents of the reactor are washed with further portions of water. Otherwise the upper product layer is removed.

The product layer is filtered using a Fisher brand 1-L membrane filter assembly with a 5-micrometer nylon filter, covered with 90 g of silica (Davison Chemical, Chromatographic Grade 62, 60 by 200 mesh (250 by 75 µm)) and 0.635 cm of magnesium sulfate, covered with glass wool. The crude filtrate is collected and chromatographed on a column (6.35 cm inner diameter, 106.68 cm in length), filled with 1200 g of silica in heptane. The excess heptane is removed from the column, which is not permitted to run dry. The effluent from the column is evaporated, using a rotary evaporator. The yield of DVS-BCB₂ can be determined at this point.

The residual product is treated with aqueous hydrogen peroxide, as above.

Chromatography on silica gel can be repeated and the heptane eluant can be removed, using a rotary evaporator.

The products are analyzed at this point by GC analysis for DVS-BCB₂, by neutron activation analysis for Cl, Br, P, Na, K and Pd and by LC analysis for higher molecular weight components.

The yield, after 26 hours heating, is 76.6 percent of DVS-BCB₂. This experiment shows that the reaction can be scaled up without decreasing the yield.

### Example 34

### Reaction of 4-BrBCB with Divinyl(dimethylsiloxane) Oligomers Using Triethylamine and Potassium Acetate in Aqueous DMF

(a) To a 1-L round-bottom flask, fitted out with a magnetic stirrer, heating mantle, reflux condenser and temperature controller, is charged 94 g of 4-BrBCB (0.51 mol), 150 g of alpha, omega-divinyl(dimethylsiloxane) oligomers (Petrarch Systems, average molecular weight 720, 0.20 mol), 58.6 g of triethylamine (0.58 mol), 42.2 g of potassium acetate (0.43 mol), 0.194 g of palladium acetate (0.864 mmol), 1.053 g of tris-(o-tolyl)phosphine (3.46 mmol), 250 mL of N,N-dimethylformamide and 125 mL of deionized water.

The contents of the flask are purged with nitrogen for 30 minutes and then heated to 92°C for 22 hours. Additional 4-BrBCB (7.5 g) is added and heating is continued. At the end of 44 hours, 7.5 g of additional 4-BrBCB and 15.0 g of triethylamine are added to the reaction mixture. Heating is continued for a total of 68 hours.

The crude reaction mixture is poured into a 2-L separatory funnel, containing 1 L of deionized water. The bottom phase is removed and toluene (200 mL) is added to the organic layer. The organic layer is washed with four 1-L aliquots of deionized water, with 50 mL of 1N HCl in 950 mL of deionized water and with four 1-L aliquots of deionized water. The organic layer is filtered through a membrane filter, packed with silica gel layer (2.54 cm) and a magnesium sulfate layer (2.54 cm). The filtrate is concentrated by a rotary evaporator at 65°C to remove most of the solvent and at 100°C under high vacuum for 2 hours. The weight of recovered product is 199.6 g.

After two days standing at room temperature trans-1,2-bis-(4-benzocyclobutenyl)ethylene precipitates and is recovered by filtration (5 g). The mother liquors are passed through a column of silica gel (4.445 cm id by 34.925 cm high), using heptane as eluant. Solvent was removed from the eluate using a rotary evaporator. The residue weighs 147 g.

The residue is charged to a 1-L round-bottom flask fitted out with a magnetic stirring bar. Heptane (150 mL) and methanol (75 mL) are charged to the flask. The mixture is stirred during the addition of 50-mL of 30 percent hydrogen peroxide in 10-mL portions over 30 minutes. The mixture is stirred during the addition of 50-mL of 30 percent hydrogen peroxide in 10-mL portions over 30 minutes. The mixture is stirred at room temperature overnight. The mixture is washed with four 1-L aliquots of deionized water and dried over magnesium sulfate. The solution is passed through a column (4.445 cm id by 46.99 cm high), packed with silica gel. The product is washed from the column with heptane. The solvent is removed using a rotary evaporator. The residue, weighing 136.7 g, is devolatilized with a wiped-film still at 100°C/70 micrometer Hg (93 Pa). The recovered bottoms fraction is devolatilized at 110°C/21 micrometer Hg (28 Pa). The product weighs 108.5 g and contains only traces of 4-BrBCB.

### Example 35

### Repeat of Example 34 With Different Molecular Weight Oligomers

The same reaction is performed, using a mixture of vinyl-terminated dimethylsiloxane oligomers (molecular weight 400 to 700, 4 to 10 dimethylsiloxane units). The product is recovered as above. The infra-red spectrum of the bis-[(4-benzocyclobutenyl]vinyl)dimethylsiloxane oligomers is:

| Frequency | Assignment |
|---|---|
| 2975 cm⁻¹ | C-H stretch, methyl, dimethylsiloxane |
| 2840 | C-H stretch, methylene, benzocyclobutene |
| 1615 | quadrant stretching mode, benzocyclobutene |
| 1585 | quadrant stretching mode, benzocyclobutene |
| 1480 | ring stretching mode, benzocyclobutene |
| 1265 | C-H symmetric deformation, Si-methyl |
| 1205 | benzocyclobutene ring stretch (C-H out of plane deformation) |
| 1030 | Si-O-Si stretch |
| 995 | trans-olefin C-H out-of-plane stretch |
| 850 | Si-methyl rocking mode |
| 805 | Si-methyl rocking mode. |

### Example 36

### Coupling of 4-BrBCB With DVS Using Low Catalyst Level, Potassium Acetate in Aqueous DMF

An experiment is run as in Example 32, except that potassium acetate (16.08 g, 0.1638 mol) is used as hydrogen halide acceptor, the catalyst comprises 0.0123 g (0.00055 mol) of palladium acetate and 0.067 g (0.00022 mol) of tris-(o-tolyl)phosphine and the solvent comprises 25 mL of DMF and 12.5 mL of deionized water. The reaction mixture is heated at 95°C for 16.5 hours at which point the mixture contains 0.8 percent of 4-vinylBCB, 3.0 percent of 4-BrBCB, 3.1 percent of DVS- BCB, 6.5 percent of trans-1,2-bis-(4- benzocyclobutenyl)ethylene and 73.1 percent of 1,1'-bis-[(4-benzocyclobutenyl)vinyl]tetramethyldisiloxane.

### Example 37

### Coupling of 4-BrBCB with DVS Using Various Hydrogen Halide Acceptors and Solvents

To a 100-mL round bottom flask, equipped with magnetic stirring bar, thermowell and reflux condenser are charged 10.0 g of 4-BrBCB, 5.08 g of DVS, 8.95 g of sodium acetate, 0.0246 g of palladium (II) acetate, 0.134 g of tris-(o-tolyl)phosphine, 25 mL of DMF and 12.5 mL of deionized water. The reaction mixture is purged with nitrogen for 30 minutes at room temperature and stirred and heated under reflux for 20 hours. At the end of 20 hours, GC analysis shows that 72 percent of the 4-BrBCB is unreacted. The reaction mixture also contains 1.0 percent of 4-vinyl BCB, 18.7 percent of DVS-BCB, and 4.1 percent of DVS-BCB₂.

At the end of 24 hours, the reaction mixture contains 61.3 percent of 4-BrBCB, 2.9 percent of vinyl BCB, 22 percent of DVS-BCB and 10.7 percent of DVS-BCB₂. At the end of 42 hours, the contents of the reaction mixture are 4.8 percent of BCB, about 4 percent DVS-BCB, 10.3 percent of bis-BCB-ethylene and 68.2 percent DVS-BCB₂.

This run corresponds to Run B:1 in Table 2.

Results of other experiments, on a laboratory scale, using similar technique but varying the hydrogen halide acceptor and solvents are shown in Table 2.

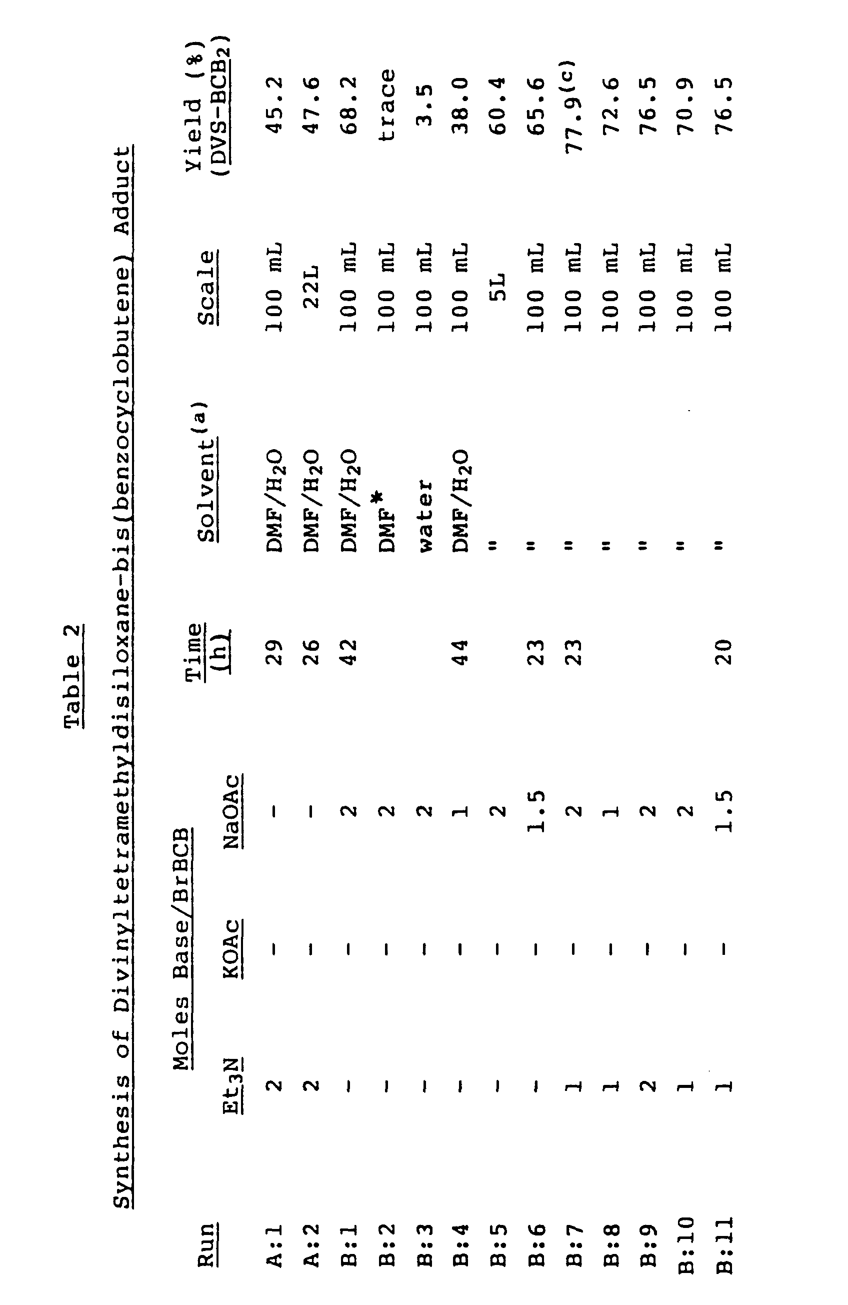

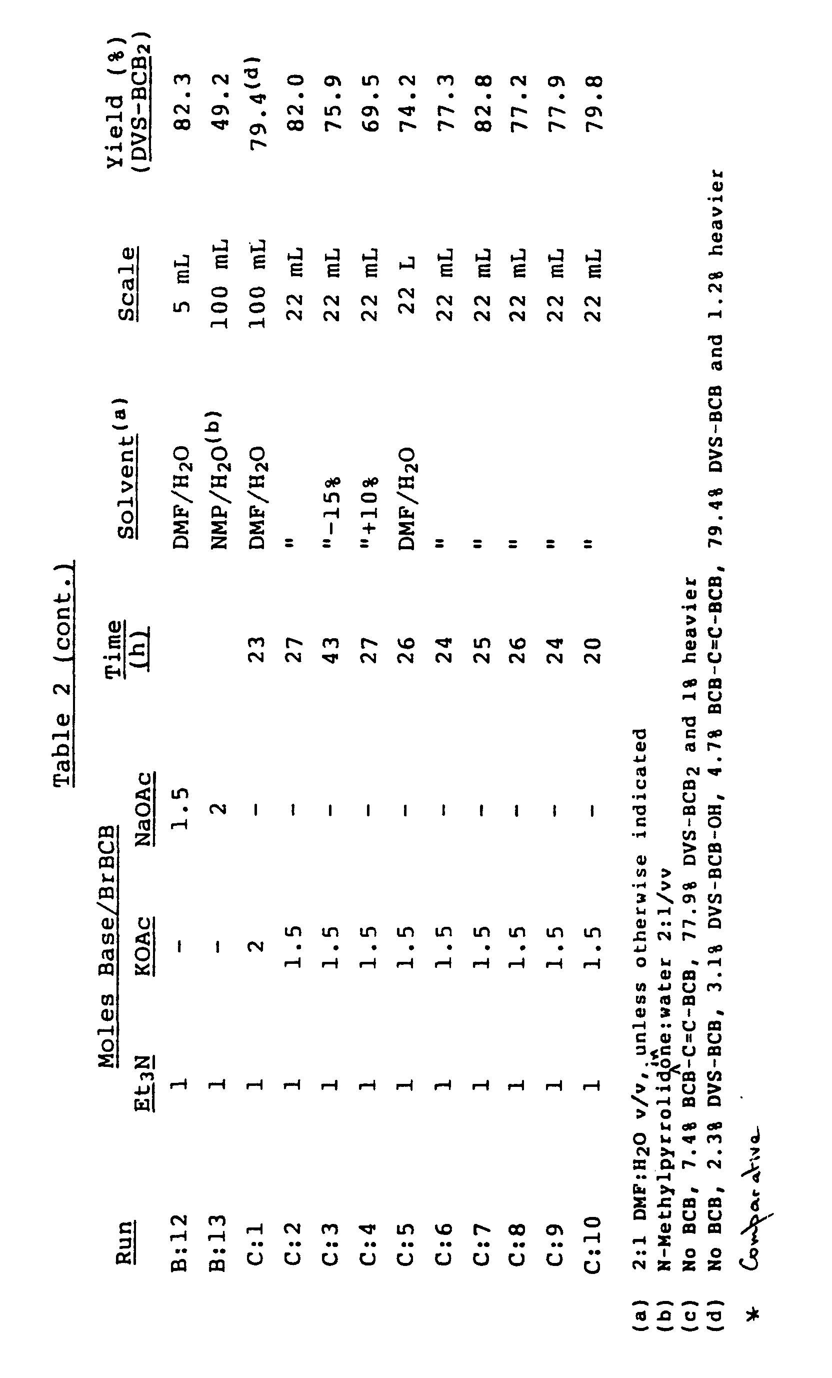

### Example 38

### Coupling of 4-BrBCB With DVS Using Lithium Acetate and Aqueous DMF

To a 100-mL one-necked flask, equipped with a polytetrafluoroethylene-coated magnetic stirrer, reflux condenser and thermometer well, is charged 12.5 mL of deionized water and 11.16 g (0.1092 mol) of lithium acetate. The mixture is stirred to dissolve the lithium acetate, after which 10.0 g (0.0546 mol) of 4-BrBCB, 5.08 g (0.0273 mol) of DVS, 0.0246 g (0.00011 mol) of palladium acetate, 0.134 g (0.00022 mol) of tris-(o-tolyl)phosphine, and 25 mL of N,N-dimethylformamide are charged to the flask. The atmosphere in the flask is replaced with nitrogen and an atmosphere of nitrogen is maintained throughout the reaction.

The mixture is stirred and heated at 93°C for 45.5 hours. The crude reaction mixture contains no 4-BrBCB, 15 percent of vinyl BCB, 1.6 percent of gem-DVS-BCB, 2.1 percent of trans-DVS-BCB, 9.6 percent of bis-BCB-ethylene, 51.5 percent of DVS-BCB₂ and 7.5 percent of higher condensates.

### Example 39

### Coupling of 4-BrBCB and DVS Using Triethylamine, Potassium Acetate and Aqueous DMF

To a 5-L, round-bottom flask, equipped with heating mantle, bottom dump valve, thermowell, reflux condenser and stirrer assembly, is charged 0.72 g of palladium acetate, 3.87 g of tris-(o-tolyl)phosphine, 290.9 g (1.59 mol) of 4-BrBCB, 591.5 g (3.18 mol) of DVS, 160.5 g of triethylamine, 234 g of potassium acetate, 436 mL of N,N-dimethyl-formamide and 219 mL of deionized water. The reactor is degassed with nitrogen and the contents heated to about 100°C and held at that temperature for two days. The crude reaction product contains no 4-BrBCB (GC), 70.55 percent of BCB-DVS and 14.69 percent of DVS-BCB₂.

The reactor is cooled to 80°C and the contents are diluted with 2000 mL of water and 500 mL of heptane. The water is separated and discarded. The organic layer is washed with three 2-L portions of deionized water. The organic phase is filtered through 150 g of silica, topped with about 40 g of magnesium sulfate, on a 5-micrometer membrane filter. Heptane is removed from the filtrate on a rotavap to give 398 g of amber liquid.

The crude product is distilled overhead on a short path molecular distillation unit at 1-2 micrometer Hg (1.3 Pa) at 80°C to give 261.46 g (57 percent isolated yield) of trans-DVS-BCB (95.47 percent purity). The bottoms cut (118 g) contains DVS-BCB, which can be redistilled.

### Example 40

### Coupling of 4-BrBCB with DVS Using Potassium Acetate and Aqueous DMF

The following reagents are used:

| | |
|---|---|
| 726 g | 4-BrBCB (3.97 mol) |
| 369 g | DVS (1.98 mol) |
| 1134 g | potassium acetate (11.56 mol) |
| 0.90 g | palladium acetate |
| 4.88 g | tris-(o-tolyl)phosphine |
| 1090 m | LN,N-dimethylformamide |
| 545 mL | deionized water |

Water is charged to the reactor and stirred. Potassium acetate is charged to the reactor and stirred until dissolved. N,N-Dimethylformamide is charged to the reactor, followed by 4-BrBCB, DVS, palladium acetate and tris-(o-tolyl)phosphine. The resulting mixture is purged with a stream of nitrogen for 30 minutes. The reaction mixture is heated to 93°C for 25 hours, at which time GC indicates that the reaction is complete.

The reaction mixture is diluted with 1000 mL of deionized water. The contents of the reactor are cooled to 60° and stirring is stopped. After phase separation has occurred, the water layer is removed and discarded.

The organic layer is diluted with 750 mL of Isopar G. The organic phase is washed with 2500-mL portions of deionized water until the aqueous wash is neutral.

### Example 41

### Removal of Phosphine Residues from Product of Example 40

The organic phase from Example 40. is stirred during the addition of 2.9 g (0.032 mol) of tert-butyl hydroperoxide. The mixture is stirred at 60°C for 16 hours and then cooled to room temperature. A filter is prepared by packing a column of suitable size with 400 g of silica gel and 90 g of magnesium sulfate, on top of a 5 micrometer filter. The organic solution is passed through the column and the column is washed with 500 mL of Isopar G. The eluate from the column is further processed as in Example 6.

Inorganic impurity content at various points of the purification procedure are:

| ppm | crude | filtered | distilled |
|---|---|---|---|
| Br | 159 | 10 | 1.9 |
| Cl | 3 | 7 | 3.3 |
| P | 305 | <2 | <0.3 |
| K | | <0.3 | |
| Na | | <0.2 | |
| Si | 8.6 | 6.3 | |

These results show that treatment of the crude product with an organic hydroperoxide produces a product, sufficiently pure for use in electronic applications.

### Example 42

### Coupling of 4-BrBCB and DVS Using Triethylamine in Acetonitrile (Comparative)

A solution of 3.0 g of 4-BrBCB, 1.52 g of DVS, 1.66 g of triethylamine, 0.152 g of tris-(o-tolyl)phosphine, 72 mg of palladium (II) acetate and 10 mL of acetonitrile in a 50 mL-round-bottom flask, equipped with a reflux condenser and magnetic stirring bar, is heated under reflux for 24 h.

At the end of 24 h, the reaction mixture is cooled to room temperature and poured into 60 mL of 10 percent aqueous hydrochloric acid. The resulting mixture is extracted with two 50-mL portions of methylene chloride and the combined methylene chloride extracts are washed with three 100-mL portions of water.

The organic phase is dried over anhydrous magnesium sulfate, filtered and evaporated in vacuo to yield a yellow oil. The oil is chromatographed on silica gel, using 20 percent toluene in heptane as eluting solvent. The eluate is evaporated to yield a colorless oil. The product is believed to be a mixture of DVS-BCB₂ and DVS-BCB.

### (Comparative) Example 43

### Reaction with Butylene Oxide With Phosphines

To a 100-mL round-bottom flask, fitted out with a magnetic stirring bar and condenser, is charged 100 g of hexane, 0.5 g (0.00164 mol) of tris-(o-tolyl)phosphine and 0.236 g (0.00328 mol) of butylene oxide. This mixture contains 99.2 percent of tris-(o-tolyl)phosphine and 0.37 percent of tris-(o-tolyl)phosphine oxide (GC analysis).

The mixture is heated at 60°C for 82 hours. The mixture contains 98.2 percent of tris-(o-tolyl)phosphine and 0.53 percent of tris-(o-tolyl)phosphine oxide.

### (Comparative) Example 44

### Reaction with Pyridine N-Oxide With Phosphines

To a 100-mL round-bottom flask, fitted out with a magnetic stirring bar and condenser, is charged 100 g of Isopar g, 0.5 g (0.00164 mol) of tris-(o-tolyl)phosphine and 0.16 g (0.00164 mol) of pyridine N-oxide. The reaction mixture contains 35.9 percent of tris-(o-tolyl)phosphine and 0.34 percent of tris-(o-tolyl)phosphine oxide (GC analysis).

The mixture is stirred and heated at 120°C for 64 hours. The reaction mixture contains 31.9 percent of tris-(o-tolyl)phosphine and 1.57 percent of tris-(o-tolyl)-phosphine oxide.

### Example 45

### Reaction with m-Chloroperoxybenzoic Acid With Phosphines

To a 100-mL round-bottom flask, fitted out with a magnetic stirring bar and reflux condenser, is charged 100 g of methylene chloride, 0.5 g (0.00164 mol) of tris-(o-tolyl)phosphine and 0.28 g (0.00164 mol) of m-chloroperoxybenzoic acid. The reaction mixture contains 37.4 percent of tris-(o-tolyl)phosphine oxide and no tris-(o-tolyl)phosphine (GC Analysis). The mixture is passed through a column packed with 5 g of silica gel. GC analysis of the eluate shows that all of the tris-(o-tolyl)phosphine oxide has been removed. The eluate is passed through a column packed with 5 g of basic alumina. GC analysis of the eluate shows that all of the m-chloroperoxybenzoic acid has been removed.

These experiments show that some oxidizing agents effectively convert a phosphine to a phosphine oxide. These experiments further show that a phosphine oxide can be adsorbed on silica gel and that a peroxidic oxidizing agent can be adsorbed on alumina.

### Example 46

Experiments are run as in Example 18, as follows:

| Olefin | Halide | Base | Solvent |
|---|---|---|---|
| VT | 4-IBCB | KOAc | DMF/water 2:1 |
| VT | 4-BrBCB | KOAc | DMF/water 1:2 |
| ST | 4-BrBCB | KOAc | MePy/water |
| VT | C₆H₅I | KOAc | DMF/water 2:1 |
| MA | C₆H₅CH₂Cl | NaOAc | NMP/water 2:1 |
| MA | C₆H₅CH₂Cl | KOAc | DMF/water 2:1 |
| AN | 4-BrBCB | LiOAc | DMF/water 1:1 |
| VT | 4-BrBCB | Bu₃N | DMF/water 2:1 v/v |
| ST | 4-BrBCB | Et₃N | DMF/water 1:1 |
| ST | 4-BrBCB | Bu₃N | DMF/water 2:1 |
| AN | 4-BrBCB | Et₃N | DMF/water 3:1 |
| VT | 4-BrBCB | Et₃N | NMP/water 2:1 |
| MA | C₆H₅CH₂Cl | Py | DMF/water 2:1 |
| AN = acrylonitrile, ST = styrene, VT = vinyltoluene, MA = methyl acrylate, KOAc = Potassium Acetate, Bu = Butyl, Et = Ethyl, Py = Pyridine | | | |

Similar results are obtained.

## Claims

1. A process for the preparation of a vinylically-unsaturated product compound, comprising reacting a halogenated organic compound with a vinylically-unsaturated precursor compound in the presence of a homogeneous zerovalent palladium catalyst complex and a hydrogen halide acceptor, **characterised in that** the reaction is conducted in a diluent selected from water and aqueous solutions containing up to 95 percent by volume of an organic solvent and providing sufficient water to dissolve hydrohalide salt formed during the reaction.

2. A process as claimed in Claim 1, wherein the reaction is conducted in a two phase system of an aqueous by-product salt-containing phase and an organic product-containing phase.

3. A process as claimed in any one of the preceding claims, wherein the diluent contains at least 25 percent by volume water.

4. A process as claimed in Claim 3, wherein the diluent contains at least 70 percent by volume water.

5. A process as claimed in Claim 1, wherein the diluent is water in an amount of 50 percent to 500 percent by weight of the combined weights of halogenated organic compound, vinylically-unsaturated precursor and organic hydrogen halide acceptor.

6. A process as claimed in Claim 5, wherein the amount of water exceeds 100 percent by weight of said combined weights.

7. A process as claimed in any one of the preceding claims, wherein the catalyst complex comprises an organophosphine or organoarsine ligand and the product is treated with a peroxide to convert organophosphine or organoarsine impurities to phosphine oxide or arsine oxide.

8. A process as claimed in any one of the preceding claims, wherein the catalyst complex is formed in situ from a palladium salt and an organophosphine or organoarsine.

9. A process as claimed in Claim 7 or Claim 8, wherein the catalyst complex is formed from a palladium salt and a triarylphosphine.

10. A process as claimed in any one of Claims 7 to 9, wherein the peroxide is aqueous hydrogen peroxide.

11. A process as claimed in any one of the preceding claims, wherein the hydrogen halide acceptor is an inorganic hydrogen halide acceptor.

12. A process as claimed in Claim 11, wherein the inorganic hydrogen halide acceptor comprises an alkali metal acetate, propionate, carbonate or hydroxide.

13. A process as claimed in any one of the preceding claims, wherein inorganic salts are formed during the reaction between the halogenated organic compound and the vinylically-unsaturated compound and sufficient water is present during said reaction to dissolve said salts.

14. A process as claimed in any one of Claims 1 to 10, wherein the hydrogen halide acceptor comprises a secondary or tertiary amine.

15. A process as claimed in any one of the preceding claims, wherein the vinylically-unsaturated precursor compound is a vinyl, allyl or methallyl organosilicon compound.

16. A process as claimed in any one of Claims 1 to 14, wherein the vinylically-unsaturated precursor compound is a hydrocarbon compound.

17. A process as claimed in any one of Claims 1 to 14, wherein the vinylically-unsaturated precursor compound is a compound containing vinyl, allyl or methallyl moieties and one or more of oxygen, nitrogen, phosphorus or sulfur atoms, or a combination thereof.

18. A process as claimed in any one of the preceding claims, wherein the diluent comprises separate aqueous and organic phases.

19. A process as claimed in any one of the preceding claims, wherein the diluent is aqueous dimethylformamide or aqueous N-methylpyrrolidinone.

20. A process as claimed in Claim 19, wherein the diluent is an aqueous solution of 30 to 70 percent by volume of dimethylformamide.

21. A process as claimed in any one of the preceding claims, wherein the halogenated organic compound is a bromo or iodo mono- or polycyclic substituted or unsubstituted carbocyclic or heterocyclic aromatic compound or a substituted or unsubstituted benzyl chloride or bromide and the vinylically-unsaturated precursor compound is a vinylically-unsaturated organosilicon precursor compound.

22. A process as claimed in any one of the preceding claims, wherein the halogenated organic compound is a bromo- or iodo- benzocyclobutene.

23. A process as claimed in Claim 22, wherein the halogenated organic compound is 4-bromobenzocyclobutene.

24. A process as claimed in Claim 22 or Claim 23, wherein the vinylically-unsaturated precursor compound is a vinyl, allyl or methallyl organosilicon compound.

25. A process as claimed in Claim 24, comprising reacting 4-bromobenzocyclobutene with a 1,1'-divinyl-tetramethyldisiloxane in the presence of (a) a homogeneous zerovalent catalyst complex, formed from palladium salt and a triarylphosphine; (b) a hydrogen halide acceptor containing potassium acetate and a diluent containing an aqueous solution of 30 to 70 percent by volume of N,N-dimethylformamide; and treating a resulting crude product mixture with aqueous hydrogen peroxide to remove organophosphine or organoarsine present in the mixture and chromatographing the thus-treated crude product over silica or alumina.

26. A process as claimed in any one of Claims 1 to 20, wherein the halogenated organic compound is a benzyl halide.

27. A process as claimed in Claim 26, wherein the vinylically-unsaturated precursor compound is selected from ethylene, styrene, vinyl toluenes and divinylbenzenes.

## Patentansprüche

1. Verfahren zur Herstellung einer vinyl-ungesättigten Produktverbindung, umfassend das Umsetzen einer halogenierten organischen Verbindung mit einer vinyl-ungesättigten Vorläuferverbindung in Anwesenheit eines homogenen nullwertigen Palladiumkatalysatorkomplexes und eines Halogenwasserstoffakzeptors, **dadurch gekennzeichnet, daß** die Reaktion in einem Verdünnungsmittel, ausgewählt aus Wasser und wässrigen Lösungen mit einem Gehalt bis zu 95 Vol.-% eines organischen Lösungsmittels, durchgeführt wird und daß ausreichend Wasser zum Auflösen von während der Reaktion gebildetem Halogenwasserstoffsalz bereitgestellt wird.

2. Verfahren nach Anspruch 1, bei welchem die Reaktion in einem Zweiphasensystem aus einer wässriges Nebenproduktsalz enthaltenden Phase und einer organisches Produkt enthaltenden Phase durchgeführt wird.

3. Verfahren nach einem der vorhergehenden Ansprüche, bei welchem das Verdünnungsmittel wenigstens 25 Vol-% Wasser enthält.

4. Verfahren nach Anspruch 3, bei welchem das Verdünnungsmittel wenigstens 70 Vol-% Wasser enthält.

5. Verfahren nach Anspruch 1, bei welchem das Verdünnungsmittel Wasser in einer Menge von 50 Gew.-% bis 500 Gew.-% der kombinierten Gewichte von halogenierter organischer Verbindung, vinyl-ungesättigtem Vorläufer und organischem Halogenwasserstoffakzeptor ist.

6. Verfahren nach Anspruch 5, bei welchem die Menge an Wasser 100 Gew.-% dieser kombinierten Gewichte übersteigt.

7. Verfahren nach einem der vorhergehenden Ansprüche, bei welchem der Katalysatorkomplex einen Organophosphin- oder Organoarsinliganden umfaßt und das Produkt mit einem Peroxid zur Umwandlung von Organophosphin- oder Organoarsinverunreinigungen zu Phosphinoxid oder Arsinoxid behandelt wird.

8. Verfahren nach einem der vorhergehenden Ansprüche, bei welchem der Katalysatorkomplex in situ aus einem Palladiumsalz und einem Organophosphin oder Organoarsin gebildet wird.

9. Verfahren nach Anspruch 7 oder Anspruch 8, bei welchem der Katalysatorkomplex aus einem Palladiumsalz und einem Triarylphosphin gebildet wird.

10. Verfahren nach einem der Ansprüche 7 bis 9, bei welchem das Peroxid wässriges Wasserstoffperoxid ist.

11. Verfahren nach einem der vorhergehenden Ansprüche, bei welchem der Halogenwasserstoffakzeptor ein anorganischer Halogenwasserstoffakzeptor ist.

12. Verfahren nach Anspruch 11, bei welchem der anorganische Halogenwasserstoffakzeptor ein Alkalimetallacetat, -propionat, -carbonat oder -hydroxid umfaßt.

13. Verfahren nach einem der vorhergehenden Ansprüche, bei welchem anorganische Salze während der Reaktion zwischen der halogenierten organischen Verbindung und der vinyl-ungesättigten Verbindung gebildet werden und ausreichend Wasser während dieser Reaktion zum Auflösen dieser Salze vorhanden ist.

14. Verfahren nach einem der Ansprüche 1 bis 10, bei welchem der Halogenwasserstoffakzeptor ein sekundäres oder tertiäres Amin umfaßt.

15. Verfahren nach einem der vorhergehenden Ansprüche, bei welchem die vinyl-ungesättigte Vorläuferverbindung eine Vinyl-, Allyl- oder Methallylorganosiliziumverbindung ist.

16. Verfahren nach einem der Ansprüche 1 bis 14, bei welchem die vinyl-ungesättigte Vorläuferverbindung eine Kohlenwasserstoffverbindung ist.

17. Verfahren nach einem der Ansprüche 1 bis 14, bei welchem die vinyl-ungesättigte Vorläuferverbindung eine Verbindung ist, welche Vinyl-, Allyl- oder Methallyleinheiten und ein oder mehrere Sauerstoff-, Stickstoff-, Phosphor- oder Schwefelatome oder eine Kombination hiervon enthält.

18. Verfahren nach einem der vorhergehenden Ansprüche, bei welchem das Verdünnungsmittel getrennte wässrige und organische Phasen umfaßt.

19. Verfahren nach einem der vorhergehenden Ansprüche, bei welchem das Verdünnungsmittel wässriges Dimethylformamid oder wässriges N-Methylpyrrolidinon ist.

20. Verfahren nach Anspruch 19, bei welchem das Verdünnungsmittel eine wässrige Lösung von 30 bis 70 Vol.-% Dimethylformamid ist.

21. Verfahren nach einem der vorhergehenden Ansprüche, bei welchem die halogenierte organische Verbindung eine brom- oder jod-, mono- oder polycyclische, substituierte oder nicht-substituierte carbocyclische oder heterocyclische aromatische Verbindung oder ein substituiertes oder nicht-substituiertes Benzylchlorid oder -bromid ist, und die vinyl-ungesättigte Vorläuferverbindung eine vinyl-ungesättigte Organosiliziumvorläuferverbindung ist.

22. Verfahren nach einem der vorhergehenden Ansprüche, bei welchem die halogenierte organische Verbindung ein Brom- oder Jodbenzocyclobuten ist.

23. Verfahren nach Anspruch 23, bei welchem die halogenierte organische Verbindung 4-Brombenzocyclobuten ist.

24. Verfahren nach Anspruch 22 oder Anspruch 23, bei welchem die vinyl-ungesättigte Vorläuferverbindung eine Vinyl-, Allyl- oder Methallylorganosiliziumverbindung ist.

25. Verfahren nach Anspruch 24, umfassend das Umsetzen von 4-Brombenzocyclobuten mit einem 1,1'-Divinyltetramethyldisiloxan in Anwesenheit von (a) einem homogenen nullwertigen Katalysatorkomplex, der aus Palladiumsalz und einem Triarylphosphin gebildet ist; (b) einem Halogenwässerstoffakzeptor, der Kaliumacetat enthält, und einem Verdünnungsmittel, das eine wässrige Lösung von 30 bis 70 Vol.-% N,N-Dimethylformamid enthält; und Behandeln eines resultierenden Rohproduktgemisches mit wässrigem Wasserstoffperoxid zur Entfernung von in dem Gemisch vorliegendem Organophosphin oder Organoarsin und Chromatographieren des so behandelten Rohproduktes über Kieselerde oder Aluminiumoxid.

26. Verfahren nach einem der Ansprüche 1 bis 20, bei welchem die halogenierte organische Verbindung ein Benzylhalogenid ist.

27. Verfahren nach Anspruch 26, bei welchem die vinyl-ungesättigte Vorläuferverbindung aus Ethylen, Styrol, Vinyltoluolen und Divinylbenzolen ausgewählt wird.

## Revendications

1. Procédé de préparation d'un produit à insaturation vinylique, comprenant le fait de faire réagir un composé organique halogéné avec un précurseur à insaturation vinylique en présence d'un complexe de catalyseur homogène à base de palladium à valence zéro et d'un accepteur d'halogénure d'hydrogène, **caractérisé par le fait que** la réaction est réalisée dans un diluant choisi parmi l'eau et les solutions aqueuses contenant jusqu'à 95 % en volume d'un solvant organique, et d'ajouter suffisamment d'eau pour dissoudre l'halogénhydrate formé en cours de réaction.

2. Procédé selon la revendication 1, dans lequel la réaction est réalisée dans un système à deux phases constitué d'une phase aqueuse contenant un produit secondaire sous forme de sel et une phase organique contenant le produit.

3. Procédé selon l'une quelconque des revendications précédentes, dans lequel le diluant contient au moins 25 % en volume d'eau.

4. Procédé selon la revendication 3, dans lequel le diluant contient au moins 70 % en volume d'eau.

5. Procédé selon la revendication 1, dans lequel le diluant est de l'eau en une quantité comprise entre 50 % et 500 % en poids rapportée au poids total du composé organique halogéné, du précurseur à insaturation vinylique et de l'accepteur d'halogénure d'hydrogène.

6. Procédé selon la revendication 5, dans lequel la quantité d'eau excède 100 % en poids rapporté audit poids total.

7. Procédé selon l'une quelconque des revendications précédentes, dans lequel le complexe de catalyseur comprend un ligand organophosphine ou organoarsine et le produit est traité avec un peroxyde afin de convertir les impuretés de type organophosphine ou organoarsine en oxyde de phosphine ou oxyde d'arsine.

8. Procédé selon l'une quelconque des revendications précédentes, dans lequel le complexe de catalyseur est formé in situ à partir d'un sel de palladium et d'une organophosphine ou organoarsine.

9. Procédé selon la revendication 7 ou 8, dans lequel le complexe de catalyseur est formé à partir d'un sel de palladium et d'une triarylphosphine.

10. Procédé selon l'une quelconque des revendications 7 à 9, dans lequel le peroxyde est du peroxyde d'hydrogène.

11. Procédé selon l'une quelconque des revendications 7 à 9, dans lequel l'accepteur d'halogénure d'hydrogène est un accepteur minéral d'halogénure d'hydrogène.

12. Procédé selon la revendication 11, dans lequel l'accepteur minéral d'halogénure d'hydrogène comprend un acétate, propionate, carbonate ou hydroxyde de métal alcalin.

13. Procédé selon l'une quelconque des revendications précédentes, dans lequel des sels minéraux se forment pendant la réaction entre le composé organique halogéné et le composé à insaturation vinylique et dans lequel suffisamment d'eau est présente pendant la réaction pour dissoudre lesdits sels.

14. Procédé selon l'une quelconque des revendications 1 à 10, dans lequel l'accepteur d'halogénure d'hydrogène comprend une amine secondaire ou tertiaire.

15. Procédé selon l'une quelconque des revendications précédentes, dans lequel le précurseur à insaturation vinylique est un composé organosilicique portant des groupes vinyle, allyle ou méthallyle.

16. Procédé selon l'une quelconque des revendications 1 à 14, dans lequel le précurseur à insaturation vinylique est un composé hydrocarboné.

17. Procédé selon l'une quelconque des revendications 1 à 14, dans lequel le précurseur à insaturation vinylique est un composé contenant des groupes vinyle, allyle ou méthallyle et un ou plusieurs atomes d'oxygène, d'azote, de phosphore ou de soufre, ou une combinaison de tels atomes.

18. Procédé selon l'une quelconque des revendications précédentes, dans lequel le diluant comprend des phases organique et aqueuse séparées.

19. Procédé selon l'une quelconque des revendications précédentes dans lequel le diluant est un diméthylformamide aqueux ou une N-méthylpyrrolidinone aqueuse.

20. Procédé selon la revendication 19, dans lequel le diluant est une solution aqueuse contenant de 30 à 70 % en volume de diméthylformamide.

21. Procédé selon l'une quelconque des revendications précédentes, dans lequel le composé organique halogéné est un composé aromatique mono- ou polycyclique, carbocyclique ou hétérocyclique, substitué ou non substitué, iodé ou bromé, ou un chlorure ou bromure de benzyle substitué ou non, et le précurseur à insaturation vinylique est un composé précurseur organosilicique à insaturation vinylique.

22. Procédé selon l'une quelconque des revendications précédentes, dans lequel le composé organique halogéné est un bromobenzocyclobutène ou un iodobenzocyclobutène.

23. Procédé selon la revendication 22, dans lequel le composé organique halogéné est le 4-bromobenzocyclobutène.

24. Procédé selon la revendication 22 ou 23, dans lequel le précurseur à insaturation vinylique est un composé organosilicique portant des groupes vinyle, allyle ou méthallyle.

25. Procédé selon la revendication 24, comprenant les étapes consistant à faire réagir du 4-bromobenzocyclobutène avec un 1,1'-divinyl-tétraméthyldisiloxane en présence (a) d'un complexe de catalyseur homogène à valence zéro, formé d'un sel de palladium et d'une triarylphosphine, (b) un accepteur d'halogénure d'hydrogène contenant de l'acétate de potassium et un diluant contenant une solution aqueuse de 30 à 70 % en volume de N,N-diméthylformamide, et à traiter le mélange brut obtenu avec du peroxyde d'hydrogène aqueux afin d'éliminer l'organophosphine ou l'organoarsine présente dans le mélange et à soumettre le produit brut ainsi traité à une chromatographie sur une colonne de silice ou d'alumine.

26. Procédé selon l'une quelconque des revendications 1 à 20, dans lequel le composé organique halogéné est un halogénure de benzyle.

27. Procédé selon la revendication 26, dans lequel le précurseur à insaturation vinylique est choisi parmi l'éthylène, le styrène, les vinyltoluènes et les divinylbenzènes.
